(19) Europäisches Patentamt
European Patent Office
Office européen des brevets

(11) EP 4 403 183 A1

(12) EUROPEAN PATENT APPLICATION

(43) Date of publication:
**24.07.2024 Bulletin 2024/30**

(21) Application number: **23382049.7**

(22) Date of filing: **23.01.2023**

(51) International Patent Classification (IPC):
***A61K 41/00*** *(2020.01)* ***A61K 47/69*** *(2017.01)*
***A61P 27/02*** *(2006.01)* ***A61P 27/04*** *(2006.01)*

(52) Cooperative Patent Classification (CPC):
(C-Sets available)
**A61K 41/0052; A61K 47/6903; A61P 27/02; A61P 27/04; G02B 1/043** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Universidade de Santiago de Compostela**
**15782 Santiago de Compostela (ES)**

(72) Inventors:
- **ÁLVAREZ LORENZO, Carmen Isabel**
  **E-15782 Santiago de Compostela, A Coruña (ES)**
- **CONCHEIRO NINE, Ángel**
  **E-15782 Santiago de Compostela, A Coruña (ES)**

(74) Representative: **ABG Intellectual Property Law, S.L.**
**Avenida de Burgos, 16D**
**Edificio Euromor**
**28036 Madrid (ES)**

# (54) GOLD NANOPARTICLE HYDROGELS

(57) The invention relates to acrylic hydrogels comprising highly anisotropic gold nanoparticles, and to methods for preparing the same. The hydrogels of the invention are characterised by a light transmittance minimum at the 532-652 nm range of the electromagnetic spectrum and photothermal responsiveness to 808 nm wavelength irradiation.

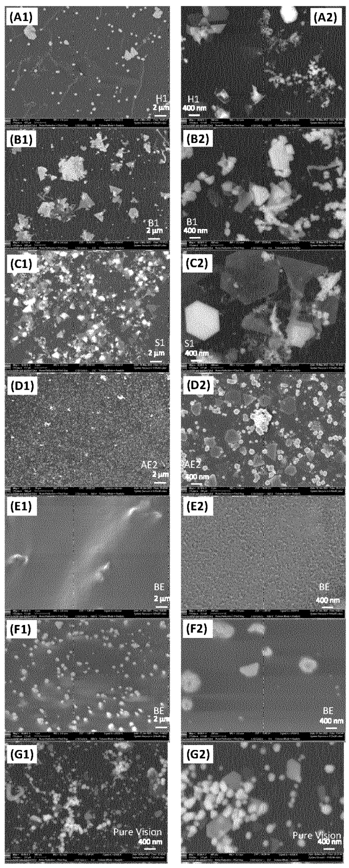


Figure 1

(52) Cooperative Patent Classification (CPC): (Cont.)

C-Sets
**G02B 1/043, C08K 3/11, C08K 3/015**

## Description

### FIELD OF THE INVENTION

This invention relates to the field of hydrogels. More particularly, the invention relates to acrylic hydrogels which incorporate gold nanoparticles and to methods for preparing such hydrogels.

### BACKGROUND

Gold nanoparticles (AuNPs) are drawing much attention in prophylaxis, therapy and diagnosis. AuNPs can endow medical devices with antibacterial and antibiofilm properties; serve as imaging agents for accurate localization or as electrochemical biosensors for *in vivo* real-time monitoring of biomarkers; are suitable for localized photothermal therapy, for instance against eye tumours on the anterior segment or for prevention and management of posterior capsule opacification associated to IOLs; and have shown beneficial effects to manage dry eye symptoms; amongst others.

A common presentation of AuNPs is in the form of a hydrogel, especially - although not exclusively - when ocular applications are desired. The hydrogels are typically applied to the eye in the form of a contact lens (CL).

Three different approaches have been tested so far to load AuNPs into CLs: (a) AuNPs are prepared in advance and then added to the CL monomers before polymerization; (b) AuNPs and CLs are prepared in separate and then the already polymerised CLs are soaked into AuNP dispersions; or (c) already polymerised CLs are soaked into a gold precursor solution and then a strong reductant chemical agent is added to trigger AuNP formation in the hydrogel bulk.

The synthetic method of choice bears great impact on the properties of the resulting hydrogel. This is true even within each of the above synthetic categories. A great part of the differing properties of said hydrogels can be attributed to the form the AuNPs adopt in the hydrogel following a particular synthetic method. It is well-known that the AuNP electric surface charge density changes as the particle morphology is varied, which leads to differing surface plasmon absorptions, and hence ultimately to different biological behaviours. AuNPs can be isotropic (exhibit just one localized surface plasmon resonance (LSPR) band), or anisotropic (different absorption bands). Whilst the earlier are typically associated to spherical AuNPs, examples of the latter are nanorods, nanotriangles, nanohexagons, nanoprisms, nanocubes, nanoarrows, nanostars, pentagons, concave rhombic dodecahedra, or icosahedrons. Each of these forms presents its own unique physiochemical behaviour.

In many instances, AuNPs are prepared by reducing Au(III) precursor compositions. The reducing agent is particularly critical in determining the final shape the AuNPs will adopt and with it the absorbance properties of the hydrogel comprising said AuNPs.

Liu et al., Gold nanoparticles-loaded contact lenses for laser protection and Meibomian Gland Dysfunction (MGD) dry eye treatment, Colloids Surf A 635 (2022) 128053 describe a method for preparing AuNP-containing hydrogels which comprises preparing gold AuNPs by inverse Turkevich method in a PVA solution and then freeze-thawing said solution to achieve physical cross-linking. The obtained AuNPs are spherical, and the hydrogels exhibit a minimum in transmittance at the approximately 521 nm wavelength and their temperature is increased by approximately 18 °C when irradiated with a 532 nm, 70 mW laser.

Liu et al., Gold nanoparticle synthesis in contact lenses for drug-less ocular cystinosis treatment, Eur. J. Pharm. Biopharm., 165 (2021), pp. 271-278 describe a method for preparing AuNP-containing hydrogels which comprises soaking a CL (i.e. the hydrogel) in both a reducing agent composition and gold precursor composition, such that these two compositions react with each other inside the CL to form the AuNPs therein. The resulting hydrogels possess a minimum in transmittance at the approximately 530 nm wavelength.

Further properties are desirable for CLs. CLs must still allow for penetration of light in the mid-region of visible spectrum to enable accurate vision after sorption of the desired radiation wavelength. Furthermore, CLs should ideally not leak AuNPs during CL wear as this can cause off-target accumulation in eye tissues and result in unwanted toxicity. Additionally, water loss from the contact lens should be minimised in order to avoid drying of the hydrogel and the resulting ocular discomfort.

A need for AuNP hydrogels satisfying the above criteria and with physiochemical properties different to those already known in the art constantly exists, such that the many and differing and ever-growing industrial applications for these materials are met and improved.

### BRIEF DESCRIPTION OF THE INVENTION

The present inventors have now unveiled AuNP hydrogels which are surprisingly highly versatile in their absorptive properties and which specifically exhibit a light transmittance minimum at the 532-652 nm range of the visible electromagnetic spectrum and increase their temperature in response to laser irradiation at different wavelengths. Equally importantly, the hydrogels of the present invention enable sharp vision through them. These properties can be partly

attributed to the highly anisotropic population of AuNPs found in said hydrogels.

Additionally, hydrogels of the present invention show no water decrease nor AuNP leakage when stored in water or a lens preservation solution for at least six months.

Thus, in a first aspect, the invention is directed to a hydrogel comprising:

- a polymer network, wherein the polymer network:

    ◦ comprises acrylic monomeric units in an amount of at least 90% by weight with respect to the total weight of the polymer network; and

    ◦ is cross-linked;

- gold nanoparticles,

wherein the hydrogel exhibits a light transmittance minimum at the 532-652 nm range within the range 500-900 nm of the electromagnetic spectrum; and

wherein the hydrogel exhibits a temperature increase of at least 7°C following irradiation with a 100 mW laser at a wavelength of 808 nm during 10 s.

The hydrogels of the invention can be attained by a method which surprisingly needs not employ external reducing agents and which affords the reduction of a gold nanoparticle precursor simply by allowing said precursor to react with the acrylic polymer network of the hydrogel. The cross-linked acrylic hydrogels of the invention show affinity for the Au metallic ion and are capable of reducing said ion within the acrylic network.

Therefore, a second aspect of the present invention relates to a method for preparing a hydrogel, comprising the steps of:

a) Providing a precursor hydrogel comprising a polymer network, wherein the polymer network:

    ◦ comprises acrylic monomeric units in an amount of at least 90% by weight with respect to the total weight of the polymer network, wherein said acrylic monomeric units comprise silicon oxide acrylic monomeric units;

    ◦ does not comprise sulphonic acid monomeric units;

    ◦ comprises, at most, 1% by weight fluorinated monomeric units with respect to the total weight of the polymer network; and

    ◦ is cross-linked;

b) Providing a gold nanoparticle precursor;

c) Reducing the gold nanoparticle precursor to gold nanoparticles, wherein the reduction comprises combining the precursor hydrogel of step a) and the gold nanoparticle precursor of step b), and is carried out in the dark, in the absence of a reducing agent and in the absence of a surfactant.

It has also unexpectedly been found that subjecting to a heat and pressure treatment, preferably autoclaving, after combining the precursor hydrogel of step a) and the gold nanoparticle precursor of step b), produces hydrogels of the invention irrespective of whether the precursor hydrogel comprises silicon oxide acrylic monomeric units or not. It has additionally been discovered that proceeding with said heat and pressure treatment enables the very rapid formation of the highly anisotropic AuNP population and can in some cases serve to tune the absorptive properties of the resulting hydrogel.

Thus, in an alternative second aspect, the invention refers to a method for preparing a hydrogel, comprising the steps of:

a) Providing a precursor hydrogel comprising a polymer network, wherein the polymer network:

    ◦ comprises acrylic monomeric units in an amount of at least 90% by weight with respect to the total weight of the polymer network; and

◦ is cross-linked;

b) Providing a gold nanoparticle precursor;

c) Reducing the gold nanoparticle precursor to gold nanoparticles, wherein the reduction:

- comprises combining the precursor hydrogel of step a) and the gold nanoparticle precursor of step b), and subjecting said combination to a heat and pressure treatment, preferably autoclaving;

- is carried out in the dark, in the absence of a reducing agent and in the absence of a surfactant.

Thus, additionally or alternatively to the above parametric definitions characterising the hydrogels of the invention (transmittance minimum and photothermal responsiveness), a hydrogel of the first aspect of the present invention can be characterised as one which is obtainable by a method according to any of these second aspects of the invention.

By virtue of their absorptive versatility, the hydrogels of the present invention are useful in a variety of industrial contexts, which are described further below. Of particular importance are the hydrogels for use in medicine.

## BRIEF DESCRIPTION OF THE DRAWINGS

Figure 1. SEM images of hydrogels of the invention. Highly anisotropic gold nanoparticle populations with a predominance of truncate triangle and hexagonal plates are immediately observable. SEM images were acquired at two magnifications (10,000x and 50,000x) for (A1, A2) H1 hydrogels, (B1, B2) B1 hydrogels, (C1, C2) S1 hydrogels, (D1, D2) AE2 hydrogels, (E1, E2) BE hydrogels before autoclaving, (F1, F2) BE hydrogels after autoclaving, and (G1, G2) Pure Vision CLs after 3 day incubation followed by autoclaving and after incubating for 45 days (without autoclaving).

Figure 2. (A, B, C, D) Transmittance of hydrogel discs after soaking in $HAuCl_4$ solution (0.327 mM, 2 mL) for 72 h and subsequent steam heat sterilization (autoclave, 121 °C, 20 min); (E) Amount of gold sorbed by the hydrogels before and after autoclaving estimated from the absorbance of the external aqueous solution. Mean values (n=3); standard deviations were below 5%.

Figure 3(A) Transmittance of commercial CLs after soaking in $HAuCl_4$ solution (0.327 mM, 2 mL) for 72 h and subsequent steam heat sterilization (autoclave, 121 °C, 20 min); (B, C, D, F) Transmittance of the commercial CLs before (0d) and after soaking in $HAuCl_4$ solution (0.327 mM, 2 mL) for 72 h (3d) and subsequent steam heat sterilization (autoclave, 121 °C, 20 min) (autoc); (E) Amount of gold sorbed by the commercial CLs before and after autoclaving estimated from the absorbance of the external aqueous solution. Mean values (n=3); standard deviations were below 5%.

Figure 4. X-ray spectra of hydrogels after soaking in $HAuCl_4$ solution (0.327 mM, 2 mL) for 72 h, subsequent steam heat sterilization (autoclave, 121 °C, 20 min), and freeze-drying. Commercial Biofinity Energys™ (BE) hydrogels were also analyzed after one month storage in the $HAuCl_4$ solution and before autoclaving. For visualization purposes, counts of X-ray spectra were progressively shifted 1,000 counts upward from sample to sample.

Figure 5. Content in water of discs and CLs measured after 3 days incubation in the $HAuCl_4$ solution (0.327 mM, 2 mL) and subsequent sterilization.

Figure 6. (A, B, C) Transmittance of S1b, S2b and S3b discs that were incubated in $HAuCl_4$ solution (0.327 mM, 2 mL) for 72 h (code "before") and then steam heat sterilized (autoclave, 121 °C, 20 min) (code "autoc"); sterilized discs were stored at room temperature protected from light during 4 days more and then the transmittance recorded again (code "autoc+4d"). (D, E, F) Evolution of the transmittance patterns of S1b, S2b and S3b discs during incubation in $HAuCl_4$ solution (0.327 mM, 2 mL) in water at room temperature protected from light for five months. Transmittance values at time 0 refer to the transmittance recorded for dried discs immediately before immersion in the gold solution. The legend refers to incubation days.

Figure 7. (A, B, C) UV-vis and near-infrared light transmittance of S1b, S2b and S3b discs as freshly synthesized (i.e. precursor hydrogel, black line), after being incubated directly in $HAuCl_4$ (0.327 mM, 2 mL) solution in water (dark intermittent line) for 143 days, and after being incubated in $HAuCl_4$ (0.327 mM, 2 mL) solution in NaCl 0.9%

for 3 days, washed with water for 5 days and then transferred to $HAuCl_4$ (0.327 mM, 2 mL) solution prepared in water and kept for 143 days (light intermittent line).

Figure 8. Relative increase in temperature recorded by a thermographic camera and decrease in illuminance recorded by a power meter sensor when the hydrogel discs were irradiated for 10 s with a green 532 nm laser pointer (top) and a NIR 808 nm laser pointer (bottom). All hydrogels were prepared by soaking in $HAuCl_4$ solution (0.327 mM, 2 mL) for 72 h, subsequent steam heat sterilization (autoclave, 121 °C, 20 min), and then storage in water for 6 months.

Figure 9. (A-C) Thermal images of wet discs before and after 10 s irradiation with green, red or NIR laser (100 mW), respectively, (D) laser light attenuation recorded during laser exposition, and (E) increase in temperature of the discs after 10 s irradiation. These hydrogels were obtained by incubation for 3 days at 20°C in $HAuCl_4$ (0.327 mM, 2 mL) solution prepared in NaCl 0.9%, then washed with water for 5 days and transferred to $HAuCl_4$ (0.327 mM, 2 mL) solution prepared in water and kept at 20°C for 143 days.

## DETAILED DESCRIPTION OF THE INVENTION

In a first aspect, the present invention is directed to hydrogels as defined above.

The term "hydrogel" refers to a three-dimensional network of polymer chains which is capable of absorbing and retaining water to form a gel in which water is the dispersion medium. Thus, a hydrogel comprises a polymer network and water, and in the context of the present invention may further comprise the AuNPs (depending on whether the precursor hydrogel or the final hydrogel is being referred to). However, weights described herein referring to the polymer network and its monomeric units refer to the weight of solely these components and do not comprise weights of other hydrogel components such as water or the AuNPs.

In an embodiment, the hydrogels of the present invention comprise water in an amount of from 20 to 80% by weight with respect to the total weight of the hydrogel.

In an embodiment, the size of the shortest dimension of the hydrogels of the present invention is greater than 1 μm. In other words, it is the gold particles, and not the hydrogel itself, which is nanoparticular. Preferably, the size of the shortest dimension of the hydrogels of the present invention is at least 5 μm, more preferably at least 10 μm, even more preferably at least 50 μm, such as from any of these values up to 10 cm, preferably up to 1 cm, more preferably up to 200 μm. The shortest dimension is typically the thickness of the hydrogel. Additionally or alternatively, the hydrogels of the present invention possess a dimension sized at least 1 mm, preferably at least 5 mm, more preferably at least 9 mm, such as from any of these values up to 100 cm, preferably up to 10 cm, more preferably up to 16 mm.

In a particularly preferred embodiment, the size of the shortest dimension of the hydrogels of the present invention is between 50 and 200 μm, and the hydrogels possess a dimension sized between 9 and 16 mm.

The polymer network comprised in the hydrogels of the present invention comprises acrylic monomeric units in an amount of at least 90% by weight with respect to the total weight of the polymer network. Preferably, said amount is at least 95%; more preferably said amount is at least 99%; and even more preferably, the polymer network does not comprise any monomeric unit different to acrylic monomeric units. In an embodiment, the polymer network consists of acrylic monomeric units. The hereinbelow amounts of the different individual acrylic monomeric units with respect to the total weight of acrylic monomeric units apply regardless of the percentage which said total acrylic monomeric units represent with respect to the total weight of the polymer network.

The term "monomeric unit", or "unit" (in the context of polymers), or "repeating unit", refers to the structural motif in a polymer that stems from a monomer that has been subjected to polymerisation. It therefore does not include any non-polymerisable compound which many end up in a non-recurring manner in a polymer chain, such as initiator molecules. It is distinguished from the monomer in that it is part of the polymer, whereas a monomer is an independent molecular entity which can be polymerised into a polymer. It is commonplace in the art to refer to monomeric units according to the structure of the monomer, even though the monomeric unit itself may no longer show exactly the same structure as the monomer. Thus, for instance, "acrylic monomeric unit" actually refers to a monomeric unit derived from an acrylic monomer by polymerisation, even though the acrylic monomeric unit no longer comprises the C=C double bond of the acrylic group that was present in the monomer, as this is the bond which has enabled the polymerisation to proceed, i.e. reacted with the incoming initiator or growing polymeric chain and then with further monomers. Similarly, ethylene oxide monomeric units do not actually comprise ethylene oxide epoxide, but refer to the unit resulting from its polymerization. The skilled person is well aware of which monomers correspond to which monomeric units. Similarly, the skilled person is well aware of how to convert monomers into corresponding monomeric units by a process of polymerization.

When the present invention refers to a monomeric unit comprising a functional group, said unit is termed [functional group] monomeric unit. For example, an acrylic monomeric unit further comprising an amine group is herein termed an amine acrylic monomeric unit.

The term "polymer", also identified by the prefix "poly", herein refers to a molecule comprising at least 10 monomeric units, such as at least 100 or at least 1000 monomeric units. Unless otherwise indicated, poly[monomeric unit X], herein refers to a molecule comprising at least 10 X monomeric units, such as at least 100 or at least 1000 X monomeric units, wherein monomeric unit X refers to a specific monomeric unit.

As used herein, the term "acrylic monomer" refers not only to acrylic acid or acrylic acid ester monomers, but also to alkylacrylic acid or alkylacrylic acid ester monomers, such as methacrylic acid or methacrylic acid ester monomers, and optionally also to vinylcarbonate analogues thereof wherein an oxygen atom is found between the carbonyl and C=C double bond group of the acrylic acid ester or alkylacrylic acid ester monomer, such as poly(dimethylsiloxy)di(silylbultanol)bis(vinyl carbamate). As used herein, the term "acrylic monomer" refers also to acrylamide and alkylacrylamide monomers such as methacrylamide monomers, and optionally also to vinylcarbamate analogues thereof wherein an oxygen atom is found between the carbonyl and C=C double bond group of the acrylamide or alkylacrylamide monomer, such as 3-[Tris(trimethylsiloxy)silyl]propyl vinyl carbamate. Preferably, the C=C double bond in the acrylic monomer is a terminal C=C double bond. The term "alkyl" refers to a straight or branched fully saturated hydrocarbon group. The alkyl is preferably a $C_{1-12}$ alkyl; more preferably a $C_{1-6}$ alkyl; even more preferably a methyl, ethyl or propyl; most preferably it is a methyl; group.

In the first aspect of the invention as described above, the hydrogels of the invention are generally characterised by their light transmittance or their photothermal responsiveness, and/or by the method by which they are prepared. Embodiments of said hydrogels can alternatively or additionally be characterised by their chemical composition.

In an embodiment, the acrylic monomeric units comprised in the polymer network comprised in the hydrogels of the present invention comprise monofunctionalized acrylic monomeric units. A monofunctionalized acrylic monomeric unit stems from a monomer containing a single acrylic group. More particularly, a polymer network comprising monofunctionalized acrylic monomeric units is the result of the polymerization of a monomer mixture comprising monomers containing a single acrylic group. In a preferred embodiment, the acrylic monomeric units comprise an amount of at least 90%; preferably of at least 99.0%; more preferably of at least 99.6%; by weight of monofunctionalized acrylic monomeric units with respect to the total weight of acrylic monomeric units. In any embodiment described herein, the maximum possible amount by weight of monofunctionalized acrylic monomeric units with respect to the total weight of acrylic monomeric units is dictated by the amount of bifunctionalized acrylic monomeric units required by said embodiment. Thus, at least 90% by weight of monofunctionalized acrylic monomeric units becomes 90% to 99.90% by weight of monofunctionalized acrylic monomeric units if the embodiment requires 0.10% by weight of bifunctionalized acrylic monomeric units.

Non-limiting examples of monofunctionalized acrylic monomeric units are 2-hydroxyethyl methacrylate (HEMA), N-(3-aminopropyl)methacrylamide (APMA), N,N-dimethylacrylamide, N,N-diethylacrylamide, methyl methacrylate or cyclohexyl methacrylate, or combinations thereof.

Preferably, the monofunctionalized acrylic monomeric units comprise alkyl or alkanol acrylic monomeric units, i.e. units wherein the non-carbonyl ester oxygen or the amide nitrogen of the acrylic group is attached to an alkyl or alkanol group, wherein the term "alkyl" is as defined above, and the term "alkanol" refers to an alkyl group as defined above substituted with an -OH group, such as HEMA monomeric units. More preferably, the monofunctionalized acrylic monomeric units comprise HEMA monomeric units. Even more preferably, at least 35%, preferably at least 85%, by weight of the monofunctionalized acrylic monomeric units, which are preferably in the above stated amounts, are alkyl or alkanol acrylic monomeric units, preferably HEMA monomeric units. The alkyl group of alkyl acrylic monomers is not to be confused with the alkyl group of alkylacrylic monomers. In alkylacrylic monomers, the alkyl group is connected to the C=C bond of the acrylic group. Thus, alkyl alkylacrylic monomers are common in the art, such as ethyl methacrylate (compound with the formula $C_2H_5O(C=O)C(CH_3)=CH_2$).

It has unexpectedly been found that when acrylic monomeric units comprised in the polymer network comprised in the hydrogels of the present invention comprise amine acrylic monomeric units, preferably APMA monomeric units, more preferably APMA monomeric units in an amount of between 0.1 and 5%, particularly of between 0.5 and 1.0%, by weight with respect to the total weight of acrylic monomeric units, then the hydrogels exhibit transmittance minimums at particularly long wavelengths, specifically at the 560-580 nm range within the range 500-900 nm of the electromagnetic spectrum.

However, in another embodiment, the acrylic monomeric units comprised in the polymer network comprised in the hydrogels of the present invention do not comprise amine acrylic monomeric units, preferably APMA monomeric units.

In an embodiment, the acrylic monomeric units comprised in the polymer network comprised in the hydrogels of the present invention comprise bifunctionalized acrylic monomeric units. A bifunctionalized acrylic monomeric unit stems from a monomer containing two or more acrylic groups. More particularly, a polymer network comprising bifunctionalized acrylic monomeric units is the result of the polymerization of a monomer mixture comprising monomers containing two or more acrylic groups. In a preferred embodiment, the acrylic monomeric units comprise an amount of between 0.01% and 10% by weight of bifunctionalized acrylic monomeric units with respect to the total weight of acrylic

monomeric units; preferably said amount is of between 0.10% and 1.0%, more preferably said amount is of between 0.15% and 0.4%, by weight of bifunctionalized acrylic monomeric units with respect to the total weight of acrylic monomeric units.

Preferably, the acrylic groups in the bifunctionalized acrylic monomeric unit are connected through an alkyl linker, wherein the term "alkyl" is as defined above. More preferably, the non-carbonyl ester oxygen or the amide nitrogen of an acrylic group is connected through an alkyl linker to the non-carbonyl ester oxygen or to the amide nitrogen of another acrylic group.

Non-limiting examples of bifunctionalized acrylic monomeric units are ethylene glycol dimethacrylate (EGDMA), 1,3-butanediol diacrylate, 1,4-butanediol diacrylate, 1,6-hexanediol diacrylate, ethylene glycol diacrylate, fluorescein O,O'-diacrylate, glycerol 1,3-diglycerolate diacrylate, pentaerythritol diacrylate monostearate, 1,6-hexanediol ethoxylate diacrylate, 3-hydroxy-2,2-dimethylpropyl 3-hydroxy-2,2-dimethylpropionate diacrylate, bisphenol A ethoxylate diacrylate, di(ethylene glycol) diacrylate, neopentyl glycol diacrylate, propylene glycol glycerolate diacrylate, tetra(ethylene glycol) diacrylate, 1,3-butanediol dimethacrylate, 1,4-butanediol dimethacrylate, 1,6-hexanediol dimethacrylate, bisphenol A dimethacrylate, diurethane dimethacrylate, fluorescein O,O'-dimethacrylate, glycerol dimethacrylate, bisphenol A ethoxylate dimethacrylate, bisphenol A glycerolate dimethacrylate, di(ethylene glycol) dimethacrylate, tetraethylene glycol dimethacrylate, tri(ethylene glycol) dimethacrylate or triethylene glycol dimethacrylate, or combinations thereof. Preferably, the bifunctionalized acrylic monomeric units comprise EGDMA monomeric units. Even more preferably, at least 35%, preferably 100%, by weight of the bifunctionalized acrylic monomeric units, which are preferably in the above stated amounts, are preferred bifunctionalized acrylic monomeric units as described above, more preferably they are EGDMA monomeric units.

In a preferred embodiment, the above amounts of monofunctionalized and bifunctionalized acrylic monomeric units are combined, preferably by their level of preference, and each amount is chosen such that the combined amount is 100% by weight with respect to the total weight of acrylic monomeric units, i.e. the acrylic monomeric units consist of monofunctionalized and bifunctionalized acrylic monomeric units. This applies to any embodiment described herein referring to a combination of monofunctionalized and bifunctionalized acrylic monomeric units.

In an embodiment, the acrylic monomeric units comprised in the polymer network comprised in the hydrogels of the present invention comprise anionic acrylic monomeric units. An anionic acrylic monomeric unit stems from an acrylic acid monomer, or an alkylacrylic acid monomer, or a monomer containing at least one acrylic group and at least one anionic group. More particularly, a polymer network comprising anionic acrylic monomeric units is the result of the polymerization of a monomer mixture comprising acrylic acid monomers, or alkylacrylic acid monomers, or monomers containing at least one acrylic group and at least one anionic group. An anionic group refers to a group with a pKa of 5 or lower, such as of between -2 and 5. Where more than one deprotonation is possible, the pKa refers to the first pKa. Examples of such groups are carboxylic acids, sulphonic acids or phosphonic acids, preferably carboxylic acids or sulphonic acids, and more preferably carboxylic acids. An anionic acrylic monomeric unit wherein the anionic group is a carboxylic acid or a sulphonic acid is herein referred to as a carboxylic or sulphonic acid anionic acrylic monomeric unit, respectively.

Preferably, the anionic acrylic monomeric units are selected from acrylic acid monomeric units or alkylacrylic acid monomeric units, or monomeric units containing at least one acrylic group and at least one anionic group wherein the anionic group is connected to the non-carbonyl ester oxygen, or the amide nitrogen, of the acrylic group, through an alkyl linker, wherein the term alkyl has the meaning defined above.

Non-limiting examples of anionic acrylic monomeric units are acrylic acid, methacrylic acid (MAA), 2-acrylamido-2-methylpropane sulfonic acid (AMPSA), methacryloyl-L-Lysine, 3-sulfopropyl methacrylate, beta-carboxyethyl acrylate, 2-sulfoethyl methacrylate and 3-sulfopropyldimethyl-3-methacrylamidopropylammonium. Most preferably, the anionic acrylic monomeric units are selected from MAA or AMPSA monomeric units. In a particular embodiment, the anionic acrylic monomeric units are MAA monomeric units. In another particular embodiment, the anionic acrylic monomeric units are AMPSA monomeric units.

In a preferred embodiment, the acrylic monomeric units comprised in the polymer network comprised in the hydrogels of the present invention comprise an amount of between 0.001% and 5% by weight of anionic acrylic monomeric units with respect to the total weight of acrylic monomeric units; more preferably said amount is of between 0.002% and 2% by weight of anionic acrylic monomeric units with respect to the total weight of acrylic monomeric units. In an embodiment, the anionic acrylic monomeric units are carboxylic acid anionic acrylic monomeric units, and are present in an amount of between 0.1% and 5% by weight with respect to the total weight of acrylic monomeric units; more preferably said amount is of between 0.5% and 2% by weight with respect to the total weight of acrylic monomeric units. In an embodiment, the anionic acrylic monomeric units are sulphonic acid anionic acrylic monomeric units, and are present in an amount of between 0.0010% and 0.0050% by weight with respect to the total weight of acrylic monomeric units; more preferably said amount is of between 0.0020% and 0.0041% by weight with respect to the total weight of acrylic monomeric units.

In another embodiment, the same amounts apply to the preferred anionic acrylic monomeric units, such as to

MAA or AMPSA monomeric units.

However, in an embodiment, the acrylic monomeric units comprised in the polymer network comprised in the hydrogels of the present invention do not comprise anionic acrylic monomeric units, more particularly sulphonic acid anionic acrylic monomeric units.

In an embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units; and
- an amount of between 0.001% and 5% by weight of anionic acrylic monomeric units, preferably in any preferred form of these as described above, more preferably MAA or AMPSA monomeric units, with respect to the total weight of acrylic monomeric units.

In further embodiments, preferred amounts for one, two or three of the three monomeric units are selected from those described above independently for each of the monomeric units. It should be noted that an anionic, aromatic or silicon oxide acrylic monomeric unit is necessarily also mono- or bi-functionalized, and therefore, the stated amount of the anionic, aromatic or silicon oxide acrylic monomeric unit is not in addition to the stated amount of mono- and bi-functionalized acrylic monomeric unit, but comprised in it. This applies throughout the present disclosure. In a preferred embodiment, the anionic, aromatic or silicon oxide acrylic monomeric unit is a monofunctionalized acrylic monomeric unit. Also, when ranges of amounts are provided for more than one of anionic, aromatic or silicon oxide acrylic monomeric units, amounts of each of these monomeric unit are chosen so as to never exceed 100% wt with respect to the total weight of acrylic monomeric units.

In a more particular embodiment, in the above embodiment:

- the monofunctionalized acrylic monomeric units consist of the MAA or AMPSA monomeric units and HEMA monomeric units;
- the bifunctionalized acrylic monomeric units are EGDMA monomeric units; and
- the anionic acrylic monomeric units are the MAA or AMPSA monomeric units.

It has unexpectedly been found that these hydrogels exhibit a light transmittance minimum at the 545-565 nm range within the range 500-900 nm of the electromagnetic spectrum.

In a particular embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units; and
- an amount of between 0.1% and 5% by weight of carboxylic acid anionic acrylic monomeric units, preferably in any preferred form of these as described above, more preferably MAA monomeric units, with respect to the total weight of acrylic monomeric units.

In further embodiments, preferred amounts for one, two or three of the three monomeric units are selected from those described above independently for each of the monomeric units. It has unexpectedly been found that, when the amount of carboxylic acid anionic acrylic monomeric units, preferably MAA monomeric units, is between 0.4% and 1.2%, preferably between 0.6% and 1.0%, by weight with respect to the total weight of acrylic monomeric units, these hydrogels exhibit a light transmittance minimum at the 545-565 nm range within the range 500-900 nm of the electromagnetic spectrum and the hydrogels also exhibit good transmittance reductions, with transmittances of between 30-50%.

In a more particular embodiment, in the above embodiment:

- the monofunctionalized acrylic monomeric units consist of the MAA monomeric units and HEMA monomeric units;
- the bifunctionalized acrylic monomeric units are EGDMA monomeric units; and
- the carboxylic acid anionic acrylic monomeric units are the MAA monomeric units.

In a particular embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units; and
- an amount of between 0.0010% and 0.0050% by weight of sulphonic acid anionic acrylic monomeric units, preferably in any preferred form of these as described above, more preferably AMPSA monomeric units, with respect to the total weight of acrylic monomeric units.

In further embodiments, preferred amounts for one, two or three of the three monomeric units are selected from those described above independently for each of the monomeric units.

In a more particular embodiment, in the above embodiment:

- the monofunctionalized acrylic monomeric units consist of the AMPSA monomeric units and HEMA monomeric units;
- the bifunctionalized acrylic monomeric units are EGDMA monomeric units; and
- the sulphonic acid anionic acrylic monomeric units are the AMPSA monomeric units.

In an embodiment, the acrylic monomeric units comprised in the polymer network comprised in the hydrogels of the present invention comprise aromatic acrylic monomeric units. An aromatic acrylic monomeric unit stems from a monomer containing at least one acrylic group and at least one aromatic group. More particularly, a polymer network comprising aromatic acrylic monomeric units is the result of the polymerization of a monomer mixture comprising monomers containing at least one acrylic group and at least one aromatic group. An aromatic group refers to a group having a cyclic, planar, conjugated pi-electron system and may be a ring system comprising 2 aromatic rings, wherein the rings may be isolated, bridged or fused. Preferably, the aromatic group comprises from 5 to 10 ring carbon atoms, such as phenyl or naphthyl, and may be substituted or unsubstituted. Preferably, the aromatic group comprises no heteroatoms. More preferably, the aromatic group is a phenyl group, which may be substituted or unsubstituted. Substitution preferably refers to fluorination or perfluorination.

Preferably, the aromatic group is connected to the non-carbonyl ester oxygen, or the amide nitrogen, of the acrylic group, either directly; through an alkyl linker, wherein the term "alkyl" has the meaning defined above; or through an alkoxy linker, wherein the term "alkoxy" refers to an -O-alkyl group, wherein "alkyl" has the meaning defined above and wherein preferably the oxygen of the -O-alkyl group is directly connected to the aromatic group. Preferably, the aromatic group is connected through an alkyl linker.

Non-limiting examples of aromatic acrylic monomeric units are benzyl methacrylate (BzMA), ethylene glycol phenyl ether methacrylate (EGPEM), pentafluorophenyl acrylate, N-benzylmethacrylamide, phenyl acrylate, phenyl methacrylate, 2-phenylethyl acrylate, 2-phenylethyl methacrylate, benzyl acrylate and 2-Hydroxy-3-phenoxypropyl methacrylate. Most preferably, the aromatic acrylic monomeric units are BzMA or EGPEM monomeric units. In a particular embodiment, the aromatic acrylic monomeric units are BzMA monomeric units. In another particular embodiment, the aromatic acrylic monomeric units are EGPEM monomeric units.

In a preferred embodiment, the acrylic monomeric units comprised in the polymer network comprised in the hydrogels of the present invention comprise an amount of between 2% and 15% by weight of aromatic acrylic monomeric units with respect to the total weight of acrylic monomeric units; more preferably said amount is of between 3% and 5% by weight of aromatic acrylic monomeric units with respect to the total weight of acrylic monomeric units.

In another embodiment, the same amounts apply to the preferred aromatic acrylic monomeric units, in particular BzMA or EGPEM monomeric units.

In an embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units; and
- an amount of between 2% and 15% by weight of aromatic acrylic monomeric units, preferably in any preferred form of these as described above, more preferably BzMA or EGPEM monomeric units, with respect to the total weight of acrylic monomeric units.

In further embodiments, preferred amounts for one, two or three of the three monomeric units are selected from those described above independently for each of the monomeric units. It has unexpectedly been found that when acrylic monomeric units comprised in the polymer network comprised in the hydrogels of the present invention comprise EPGEM monomeric units, especially in the above provided amounts, then the hydrogels exhibit transmittance minimums at particularly long wavelengths, specifically at the 560-580 nm range within the range 500-900 nm of the electromagnetic spectrum.

In a more particular embodiment, in the above embodiment:

- the monofunctionalized acrylic monomeric units consist of the BzMA or EGPEM monomeric units and HEMA monomeric units;
- the bifunctionalized acrylic monomeric units are EGDMA monomeric units; and
- the aromatic monomeric units are the BzMA or EGPEM monomeric units.

In an embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units;
- an amount of between 0.1% and 5% by weight of carboxylic acid anionic acrylic monomeric units, preferably in any preferred form of these as described above, more preferably MAA monomeric units, with respect to the total weight of acrylic monomeric units;
- an amount of between 2% and 15% by weight of aromatic acrylic monomeric units, preferably in any preferred form of these as described above, more preferably BzMA monomeric units, with respect to the total weight of acrylic monomeric units.

In further embodiments, preferred amounts for one, two, three or four of the four monomeric units are selected from those described above independently for each of the monomeric units. It has unexpectedly been found that, when the amount of carboxylic acid anionic acrylic monomeric units, preferably MAA, is between 0.4% and 1.2%, preferably between 0.6% and 1.0%, by weight with respect to the total weight of acrylic monomeric units, these hydrogels exhibit a light transmittance minimum at the 545-565 nm range within the range 500-900 nm of the electromagnetic spectrum and the hydrogels also exhibit good transmittance reductions, with transmittances of between 40-50%.

In a more particular embodiment, in the above embodiment:

- the monofunctionalized acrylic monomeric units consist of the MAA monomeric units, the BzMA monomeric units, and HEMA monomeric units;
- the bifunctionalized acrylic monomeric units are EGDMA monomeric units;
- the carboxylic acid anionic acrylic monomeric units are the MAA monomeric units; and
- the aromatic monomeric units are the BzMA monomeric units.

In an embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units;
- an amount of between 0.0010% and 0.0050% by weight of sulphonic acid anionic acrylic monomeric units, preferably in any preferred form of these as described above, more preferably AMPSA monomeric units, with respect to the total weight of acrylic monomeric units; and
- an amount of between 2% and 15% by weight of aromatic acrylic monomeric units, preferably in any preferred form of these as described above, more preferably BzMA monomeric units, with respect to the total weight of acrylic monomeric units.

In further embodiments, preferred amounts for one, two, three or four of the four monomeric units are selected

from those described above independently for each of the monomeric units.

In a more particular embodiment, in the above embodiment:

- the monofunctionalized acrylic monomeric units consist of the AMPSA monomeric units, the BzMA monomeric units, and HEMA monomeric units;
- the bifunctionalized acrylic monomeric units are EGDMA monomeric units;
- the sulphonic acid anionic acrylic monomeric units are the AMPSA monomeric units; and
- the aromatic monomeric units are the BzMA monomeric units.

In a preferred embodiment, the acrylic monomeric units comprised in the polymer network comprised in the hydrogels of the present invention comprise silicon oxide acrylic monomeric units. These hydrogels are herein also referred to as S-series hydrogels. A silicon oxide acrylic monomeric unit stems from a monomer containing at least one acrylic group and at least one silicon oxide group. More particularly, a polymer network comprising silicon oxide acrylic monomeric units is the result of the polymerization of a monomer mixture comprising monomers containing at least one acrylic group and at least one silicon oxide group. A silicon oxide group refers to a group comprising a Si-O linkage.

Preferably, a Si atom in the silicon oxide group is connected to the non-carbonyl ester oxygen, or the amide nitrogen, of the acrylic group through an alkyl linker, wherein the term "alkyl" has the meaning defined above.

Alternatively or additionally, in a preferred embodiment, the silicon oxide group comprises at least one, such as up to six, preferably up to three, terminal -($OSiR'_3$) groups, wherein each R' is independently or simultaneously H or an alkyl group as described above, and preferably an alkyl group as described above. A preferred example of such monomeric unit is the 3-[Tris(trimethylsiloxy)silyl]propyl methacrylate (aka TRIS) unit.

Alternatively or additionally, in another preferred embodiment, the silicon oxide group comprises a non-terminal

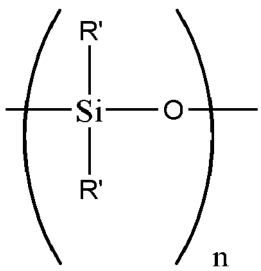

group, wherein each R' is independently or simultaneously H or an alkyl group as described above, preferably an alkyl group as described above; and n is an integer number from 1 to 30, more preferably 4 to 20 (aka silicone). Particularly preferred are silicone acrylic monomeric units resulting from polymerization of acrylic monomers of the following formulae:

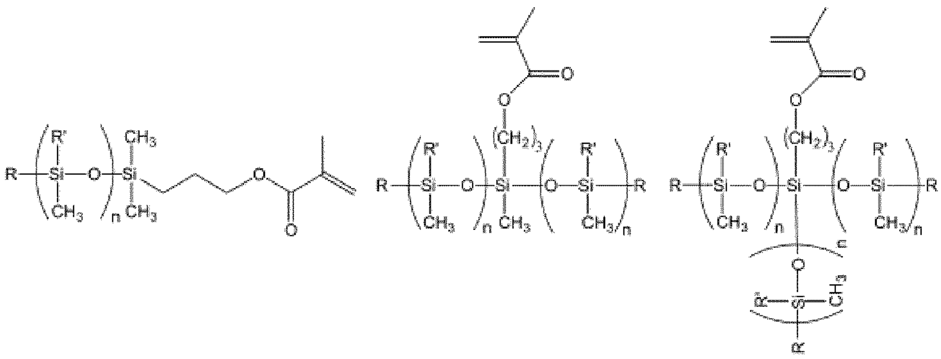

**Asymmetric (R)** **Symmetric (S)** **T-Structure (T)**

wherein each R' and n independently have the above indicated meaning, and each R independently or simultaneously is methyl, n-butyl, $HO(CH_2)_3$- or $CH_3O(CH_2)_3$-.

Non-limiting examples of silicon oxide acrylic monomeric units are monomethacryloxypropyl-sym-polydimethylsiloxane hydroxypropyl terminated (MCS-MC12), MCR-M11, MFR-M15, MCR-ME11, MCS-M11, MFS-M15, MCS-ME11, MCT-M11 (acronyms as described in Goff et al., Living Polymerization Routes to Siloxane Macromers and Higher Order Silicone Structures, Progress in Silicones and Silicone-Modified Materials ed S. Clarson 2013, Chapter 5, 59-78),

(3-Methacryloxy-2-hydroxypropoxy)propylbis(trimethylsiloxy)methylsilane, 3-acrylamidopropyltrimethoxysilane, 3-acrylamidopropyltris(trimethylsiloxy)silane, (2-acryloxyethoxy)trimethylsilane, n-(3 -acryloxy-2-hydroxypropyl)-3 - aminopropyltriethoxysilane, acryloxymethyltrimethoxysilane, (acryloxymethyl)phenethyltrimethoxysilane, acryloxymethyltrimethylsilane, (3-acryloxypropyl)methylbis(trimethylsiloxy)silane, (3-acryloxypropyl)methyldimethoxysilane, (3-acryloxypropyl)tris(trimethylsiloxy)silane, methacryloxypropyltrimethoxysilane, methacryloxypropyl terminated polydimethylsiloxane. Most preferably, the silicon oxide acrylic monomeric units are MCS-MC12, MCR-M11, MFR-M15, MCR-ME11, MCS-M11, MFS-M15, MCS-ME11 or MCT-M11 units, yet more preferably they are MCS-MC12 units.

In a preferred embodiment, the acrylic monomeric units comprised in the polymer network comprised in the hydrogels of the present invention comprise an amount of between 2% and 90%, preferably between 2% and 65%, by weight of silicon oxide acrylic monomeric units with respect to the total weight of acrylic monomeric units; preferably said amount is of between 10% and 50%, more preferably of between 10 and 15%, even more preferably of between 12% and 13%, by weight of silicon oxide acrylic monomeric units with respect to the total weight of acrylic monomeric units.

In another embodiment, the same amounts apply to the preferred silicon oxide acrylic monomeric units, and more particularly to the MCS-MC12, MCR-M11, MFR-M15, MCR-ME11, MCS-M11, MFS-M15, MCS-ME11 or MCT-M11 units; even more particularly to the MCS-MC12 units.

In an embodiment, in any embodiment described herein referring to silicon oxide acrylic monomeric units, the elemental Si content of the hydrogel surface is at least 1%, preferably at least 4%, such as from any of said values up to 15%, preferably up to 10%. Elemental Si content may be measured by X-ray photoelectron spectroscopy (XPS) as described in Karlgard et al., Applied Surface Science 230 (2004) 106-114 (drying in nitrogen).

In a preferred embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units; and
- an amount of between 2% and 90%, preferably between 2% and 65%, by weight of silicon oxide acrylic monomeric units, preferably in any preferred form of these as described above, more preferably TRIS monomeric units, with respect to the total weight of acrylic monomeric units.

In further embodiments, preferred amounts for one, two or three of the three monomeric units are selected from those described above independently for each of the monomeric units.

In a preferred embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units; and
- an amount of between 2% and 15% by weight of silicon oxide acrylic monomeric units, preferably in any preferred form of these as described above, more preferably MCS-MC12 monomeric units, with respect to the total weight of acrylic monomeric units.

In further embodiments, preferred amounts for one, two or three of the three monomeric units are selected from those described above independently for each of the monomeric units. It has unexpectedly been found that these hydrogels exhibit a light transmittance minimum at the 540-575 nm range within the range 500-900 nm of the electromagnetic spectrum and the hydrogels also exhibit excellent transmittance reductions, with transmittances of between 5-50%. In a very preferred embodiment, the acrylic monomeric units do not comprise anionic monomeric units, and more preferably do not comprise monomeric units other than the three described above. It has unexpectedly been found that such hydrogels can reach particularly outstanding transmittance reductions when prepared without the heat and pressure treatment, with transmittances of between 5-15%. It has unexpectedly also been observed that such hydrogels provide large temperature increases when irradiated with a 100 mW laser at a wavelength of 532 nm or 808 nm during 10 s.

In a more particular embodiment, in the above embodiment:

- the monofunctionalized acrylic monomeric units consist of the MCS-MC12 monomeric units and HEMA monomeric units;

- the bifunctionalized acrylic monomeric units are EGDMA monomeric units; and
- the silicon oxide acrylic monomeric units are the MCS-MC12 monomeric units.

In another very preferred embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units;
- an amount of between 0.1% and 5% by weight of carboxylic acid anionic acrylic monomeric units, preferably in any preferred form of these as described above, more preferably MAA monomeric units, with respect to the total weight of acrylic monomeric units;
- an amount of between 2% and 15% by weight of silicon oxide acrylic monomeric units, preferably in any preferred form of these as described above, more preferably MCS-MC12 monomeric units, with respect to the total weight of acrylic monomeric units.

In further embodiments, preferred amounts for one, two, three or four of the four monomeric units are selected from those described above independently for each of the monomeric units. It has unexpectedly been found that these hydrogels can exhibit transmittance minimums at particularly long wavelengths, specifically at the 560-605 nm range within the range 500-900 nm of the electromagnetic spectrum, when the preparation of the hydrogel does not comprise the heat and pressure treatment. Furthermore, such hydrogels exhibit a particularly outstanding temperature increase following irradiation with a 100 mW laser at a wavelength of 808 nm during 10 s, of 23-25°C.

The wavelength shift becomes more pronounced with increasing amounts of carboxylic acid anionic acrylic monomeric units, preferably MAA monomeric units; thus, in a preferred embodiment, the carboxylic acid anionic acrylic monomeric units, preferably MAA monomeric units, are comprised in an amount of between 1.5% and 5% by weight with respect to the total weight of acrylic monomeric units. Furthermore, such hydrogels exhibit a particularly outstanding temperature increase following irradiation with a 100 mW laser at a wavelength of 532 nm during 10 s, of 65-67°C.

In a more particular embodiment, in the above embodiment:

- the monofunctionalized acrylic monomeric units consist of the MAA monomeric units, the MCS-MC12 monomeric units and HEMA monomeric units;
- the bifunctionalized acrylic monomeric units are EGDMA monomeric units;
- the carboxylic acid anionic acrylic monomeric units are the MAA monomeric units; and
- the silicon oxide acrylic monomeric units are the MCS-MC12 monomeric units.

In an embodiment, the acrylic monomeric units comprised in the polymer network comprised in the hydrogels of the present invention comprise:

- an amount of at least 90% by weight of monofunctionalized acrylic monomeric units, preferably in any preferred form of these as described above, more preferably out of which at least 85% wt are HEMA monomeric units; and an amount of between 0.01% and 10% by weight of bifunctionalized acrylic monomeric units, preferably in any preferred form of these as described above, more preferably EGDMA monomeric units; with respect to the total weight of acrylic monomeric units
- an amount of between 0.0010% and 0.0050% by weight of sulphonic acid anionic acrylic monomeric units, preferably in any preferred form of these as described above, more preferably AMPSA monomeric units, with respect to the total weight of acrylic monomeric units; and
- an amount of between 2% and 15% by weight of silicon oxide acrylic monomeric units, preferably in any preferred form of these as described above, more preferably MCS-MC12 monomeric units, with respect to the total weight of acrylic monomeric units.

In further embodiments, preferred amounts for one, two, three or four of the four monomeric units are selected from those described above independently for each of the monomeric units.

In a more particular embodiment, in the above embodiment:

- the monofunctionalized acrylic monomeric units consist of the AMPSA monomeric units, the MCS-MC12 monomeric units and HEMA monomeric units;

- the bifunctionalized acrylic monomeric units are EGDMA monomeric units;
- the sulphonic acid anionic acrylic monomeric units are the AMPSA monomeric units; and
- the silicon oxide acrylic monomeric units are the MCS-MC12 monomeric units.

The following disclaimers apply to any of the hydrogel embodiments described herein.

In an embodiment, the hydrogel of the invention does not comprise a further polymer in addition to the polymeric network comprised in said hydrogel.

In an embodiment, the hydrogel of the invention or the polymer network comprised in the hydrogel of the present invention does not comprise vinylic monomeric units, preferably vinylpyrrolidone, vinylalcohol or propyleneimine monomeric units, more preferably vinylpyrrolidone monomeric units. Alternatively, the hydrogel of the invention or the polymer network comprised in the hydrogel of the present invention comprises these monomeric units in an amount of at most 1%, preferably at most 0.1%, by weight with respect to the total weight of the hydrogel or polymer network, respectively.

In an embodiment, the hydrogel of the invention or the polymer network comprised in the hydrogel of the present invention does not comprise amine monomeric units such as ethyleneimine, propyleneimine, allylamine, aniline, N,N-dimethylaminoethyl methacrylate (DMAEMA), phenylenediamine, anisidine, aminophenol or pyrrole monomeric units, more preferably non-acrylic amine monomeric units such as any of those recited immediately above. In a preferred embodiment, the hydrogel of the invention or the polymer network comprised in the hydrogel of the present invention does not comprise DMAEMA monomeric units. Alternatively, the hydrogel of the invention or the polymer network comprised in the hydrogel of the present invention comprises these monomeric units in an amount of at most 1%, preferably at most 0.1%, by weight with respect to the total weight of the hydrogel or polymer network, respectively.

In an embodiment, the hydrogel of the invention or the polymer network comprised in the hydrogel of the present invention does not comprise alkylene oxide monomeric units, preferably ethylene oxide or propylene oxide monomeric units. Alternatively, the hydrogel of the invention or the polymer network comprised in the hydrogel of the present invention comprises these monomeric units in an amount of at most 1%, preferably at most 0.1%, by weight with respect to the total weight of the hydrogel or polymer network, respectively. In a preferred embodiment, the gold nanoparticles comprised in the hydrogels of the present invention do not comprise, and more specifically are not coated with, alkylene oxide monomeric units, preferably ethylene oxide monomeric units.

In an embodiment, the hydrogel of the invention or the polymer network comprised in the hydrogel of the present invention does not comprise saccharide monomeric units, preferably glucose, galactose, arabinose, rhamnose, glucuronic acid monomeric units. Alternatively, the hydrogel of the invention or the polymer network comprised in the hydrogel of the present invention comprises these monomeric units in an amount of at most 1%, preferably at most 0.1%, by weight with respect to the total weight of the hydrogel or polymer network, respectively.

In an embodiment, the hydrogel of the invention or the polymer network comprised in the hydrogel of the present invention does not comprise non-acrylic Si-containing monomeric units, preferably non-acrylic siloxane monomeric units, more preferably non-acrylic dimethylsiloxane monomeric units. A non-acrylic Si-containing monomeric unit refers to a monomeric unit which is connected to the polymer backbone not through an acrylic group. Alternatively, the hydrogel of the invention or the polymer network comprised in the hydrogel of the present invention comprises these monomeric units in an amount of at most 1%, preferably at most 0.1%, by weight with respect to the total weight of the hydrogel or polymer network, respectively.

In an embodiment, the hydrogel of the invention or the polymer network comprised in the hydrogel of the present invention does not comprise non-acrylic anionic monomeric units, preferably non-acrylic carboxylic acid monomeric units, more preferably citric acid monomeric units. Alternatively, the hydrogel of the invention or the polymer network comprised in the hydrogel of the present invention comprises these monomeric units in an amount of at most 1%, preferably at most 0.1%, by weight with respect to the total weight of the hydrogel or polymer network, respectively.

In an embodiment, the hydrogel of the invention or the polymer network comprised in the hydrogel of the present invention comprises at most 1% by weight fluorinated monomeric units with respect to the total weight of the hydrogel or polymer network, respectively, or does not comprise fluorinated monomeric units, as these have been found to substantially decrease the formation of AuNPs.

In a preferred embodiment, the hydrogel of the invention does not comprise surfactant. As is explained hereinbelow, the method of the invention does not require the use of a surfactant in the formation of the anisotropic gold nanoparticles. In a more particular embodiment, the gold nanoparticles do not comprise, and more specifically are not coated with, a surfactant. In any of these embodiments, the excluded surfactant is selected from the list provided hereinbelow for the method of the invention and is preferably CTAB.

In a preferred embodiment, the hydrogel of the invention does not comprise silver.

The above disclaimers are to be viewed independently and also in combination. Any individualised combination of two or more disclaimers is covered by the present invention. Particularly preferred combinations are the above disclaimers relating to:

- surfactant and alkylene oxide monomeric units optionally further silver; this disclaimer combination is furthermore preferably combined with the above embodiments relating to the size of the hydrogel of the invention.
- surfactant and amine monomeric units.

The polymer network comprised in the hydrogels of the invention is cross-linked. Cross-linking may be achieved by different manners known to the skilled person, such as by chemical means, such as by the inclusion of bifunctionalized acrylic monomers in the polymerisation reaction. Bifunctionalized acrylic monomers are monomers which are polymerised into bifunctionalized acrylic monomeric units. These monomeric units were described above. In an embodiment, cross-linking is not achieved by freeze-thawing.

The presence of and crystallinity of the gold nanoparticles comprised in the hydrogels of the present invention can be defined through the X-ray spectra of the hydrogels of the invention. Thus, the hydrogels of the present invention are characterised by an X-ray powder diffraction pattern measured using Cu $K_{\alpha 1}$ radiation at a wavelength of 1.5406 Å comprising a peak at diffraction angle 2θ of 38.078 ± 1.0°. Preferably, said pattern comprises further peaks at 44.256 ± 1.0° and 64.376 ± 1.0°. The first peak is greater in intensity to the two further peaks, and more preferably, the first peak is the most intense peak in the pattern (0 to 70°). These three peaks correspond to Au face center cubic lattice nanocrystals. The same X-ray powder diffraction pattern is further characterised by broader bands in the 10-30° range. These bands are representative of the amorphous state of the polymer network. Further detail regarding the X-ray powder diffraction pattern method of measurement is provided in Example 5 hereinbelow.

The gold nanoparticles comprised in the hydrogels of the present invention are highly anisotropic. This can be easily observed in Figure 1. More specifically, the hydrogels of the present invention comprise a population of gold nanoparticles wherein truncate triangle and hexagonal plates predominate over other gold nanoparticle morphologies. Therefore, in a particular embodiment, the hydrogels of the invention comprise gold nanoparticles in the form of triangle plates, in particular truncate triangle plates. In another particular embodiment, the hydrogels of the invention comprise gold nanoparticles in the form of hexagonal plates. In another particular embodiment, the hydrogels of the invention comprise gold nanoparticles in the form of triangle and hexagonal plates, in particular truncate triangle and hexagonal plates. In further embodiments, the number of gold nanoparticles exhibiting these shapes is greater to the number of gold nanoparticles of any other one gold nanoparticle morphology.

Alternatively or additionally, the high anisotropicity of the gold nanoparticle population comprised in the hydrogels of the present invention can be defined by its mean circularity. In an embodiment, the gold nanoparticles exhibit a mean circularity value of less than 0.85, such as between 0.60 and 0.85, more particularly between 0.65 and 0.84. In an embodiment, the term "mean" refers to a mean of at least 15 particles, preferably of at least 50 particles, more preferably of at least 100 particles, such as from any of these values up to 150 particles. The term circularity (C) has its conventional meaning in the field of image analysis [Wojnar, L. et al., Practical Guide to Image Analysis, ASM International, 2000, p 157-160] and is herein calculated for each individual particle applying the formula

$$C = 4\pi A / P^2$$

where A is the two-dimensional projection area of the form and P is the two-dimensional projection perimeter of the form. The area (A) and perimeter (P) values can be directly taken from the values provided by ImageJ software for SEM images of the gold nanoparticles, as described in detail in Example 5 hereinbelow. Accordingly, a perfect circle (i.e. a two dimensional projection of a spherical gold nanoparticle) has a theoretical circularity value of 1, and any other geometrical form has a circularity of less than 1; for instance a hexagon typically has a circularity value of 0.84-0.90, whereas an equilateral triangle has a circularity value of 0.55-0.60. The circularity of all particles analysed in each image can be expressed as a mean value.

The gold nanoparticles comprised in the hydrogels of the present invention exhibit a particle size from 1000 nm to 10 nm. The mean particle size of the gold nanoparticles ranges from 100 to 400 nm, preferably from 150 to 350 nm. In particular, said size corresponds to the Feret diameter of the nanoparticle, the Feret diameter corresponding to the distance of the longest line that can be drawn through the particle, also known as maximum caliper diameter. Values were directly taken from ImageJ software for SEM images of the gold nanoparticles, as described in detail in Example 5 hereinbelow. The size of all particles analyzed in each image was expressed as a mean value. In an embodiment, the term "mean" refers to a mean of at least 15 particles, preferably of at least 50 particles, more preferably of at least 100 particles, such as from any of these values up to 150 particles.

Alternatively or additionally, the hydrogels of the present invention can be characterised by their light transmittance spectrum, more particularly by their localized surface plasmon resonance transmittance. Specifically, the hydrogels of the present invention exhibit a transmittance minimum (i.e. the point of the spectrum showing the lowest transmittance value) at the 532-652 nm range, preferably at the 535-605 nm range, more preferably at the 540-605 nm range, within the range 500-900 nm of the electromagnetic spectrum. Preferably, said minimum does not extend outside these nm

ranges.

In a preferred embodiment, the transmittance minimum is of 60% transmittance or lower, more preferably of 50% transmittance or lower, even more preferably of 40% transmittance or lower, such as from any of these values down to 10% transmittance, or from any of these values down to 20% transmittance.

Further detail regarding transmittance method of measurement is provided in Example 3 hereinbelow.

Alternatively or additionally, the hydrogels of the present invention are characterised by their photothermal responsiveness. Specifically, the hydrogels of the present invention exhibit a temperature increase of at least 7°C, such as of at least 7.6°C, following irradiation with a 100 mW laser at a wavelength of 808 nm during 10 s. The temperature increase can range from any of said values up to 25°C, such as up to 24.6°C.

Alternatively or additionally, the hydrogels of the present invention exhibit a temperature increase of at least 9°C, such as of at least 9.6°C following irradiation with a 100 mW laser at a wavelength of 532 nm during 10 s. The temperature increase can range from any of said values up to 67°C, such as up to 66.4°C.

In a preferred embodiment, the polymer network comprised in the hydrogel of the invention:

◦ comprises acrylic monomeric units in an amount of at least 90% by weight with respect to the total weight of the polymer network, wherein said acrylic monomeric units comprise silicon oxide acrylic monomeric units, as described in any embodiment described hereinabove;

◦ does not comprise sulphonic acid monomeric units;

◦ comprises, at most, 1% by weight fluorinated monomeric units with respect to the total weight of the polymer network.

These hydrogels exhibit a temperature increase of at least 14°C, such as of at least 15.1°C, following irradiation with a 100 mW laser at a wavelength of 808 nm during 10 s. The temperature increase can range from any of said values up to 25°C, such as up to 24.6°C. These hydrogels exhibit a temperature increase of at least 46°C, such as of at least 47.5°C, following irradiation with a 100 mW laser at a wavelength of 532 nm during 10 s. The temperature increase can range from any of said values up to 67°C, such as up to 66.4°C. These hydrogels exhibit a temperature increase of at least 27°C, such as of at least 28.3°C, following irradiation with a 100 mW laser at a wavelength of 652 nm during 10 s. The temperature increase can range from any of said values up to 30°C, such as up to 29.4°C. Such hydrogels are attainable when prepared according to a method of the invention not comprising the heat and pressure treatment.

Further detail regarding the method of measurement of temperature increase following laser irradiation is provided in Example 4 hereinbelow.

The second aspect of the present invention relates to a method for preparing the hydrogels of the invention.

When the hydrogel comprises silicon oxide acrylic monomeric units and no sulphonic acid anionic acrylic monomeric units, the heat and pressure treatment of the hydrogel is not necessary. Thus, in an embodiment, the method of the invention comprises the steps of:

a) Providing a precursor hydrogel comprising a polymer network, wherein the polymer network:

◦ comprises acrylic monomeric units in an amount of at least 90% by weight with respect to the total weight of the polymer network, wherein said acrylic monomeric units comprise silicon oxide acrylic monomeric units;

◦ does not comprise sulphonic acid anionic acrylic monomeric units;

◦ comprises, at most, 1% by weight fluorinated monomeric units with respect to the total weight of the polymer network; and

◦ is cross-linked;

b) Providing a gold nanoparticle precursor;

c) Reducing the gold nanoparticle precursor to gold nanoparticles, wherein the reduction comprises combining the precursor hydrogel of step a) and the gold nanoparticle precursor of step b) and is carried out in the dark, in the absence of a reducing agent and in the absence of a surfactant.

This method will hereinafter be referred to as Method 1.

When the hydrogel of the invention does not comprise silicon oxide acrylic monomeric units, the heat and pressure treatment of the hydrogel is generally necessary in order to achieve reduction of the gold nanoparticle precursor

to gold nanoparticles within the precursor hydrogel in such a manner that the resulting hydrogel possesses the herein described light transmittance and photothermal responsiveness. Thus, in an embodiment, the method of the invention comprises the steps of:

a) Providing a precursor hydrogel comprising a polymer network, wherein the polymer network:

◦ comprises acrylic monomeric units in an amount of at least 90% by weight with respect to the total weight of the polymer network; and
◦ is cross-linked;

b) Providing a gold nanoparticle precursor;
c) Reducing the gold nanoparticle precursor to gold nanoparticles, wherein the reduction:

- comprises combining the precursor hydrogel of step a) and the gold nanoparticle precursor of step b), and subjecting said combination to a heat and pressure treatment;

- is carried out in the dark, in the absence of a reducing agent and in the absence of a surfactant.

This method will hereinafter be referred to as Method 2.

Step a) in either case requires providing the precursor hydrogel. As used herein, "precursor hydrogel" refers to the hydrogel which does not comprise gold nanoparticles. The precursor hydrogel of the present invention can be prepared by methods of monomer mixture polymerisation well-known to the person skilled in the art. It is understood that the monomers in the mixture must be polymerizable, i.e. they must comprise a functional group which can react with other monomers in a polymerization reaction. For instance, polymer networks comprising acrylic monomeric units are produced from a mixture of acrylic monomers, i.e. monomers comprising at least one acrylic group, which comprises the polymerizable C=C double bond. Such monomers are readily commercially available, as described in the Examples.

Every embodiment provided herein describing the nature and amounts of the monomeric units comprised in the polymer network comprised in the hydrogels of the present invention applies *mutatis mutandis* to the precursor hydrogel. Similarly, the person skilled in the art knows how to translate the composition of said hydrogels into the corresponding non-polymerised monomer mixture.

In an embodiment, every embodiment provided herein describing the nature and amounts of the monomeric units comprised in the polymer network comprised in the hydrogels of the present invention refers rather to the nature and amounts of the corresponding monomers in the non-polymerised monomer mixture that is to be subjected to polymerisation. Thus, by way of example, when the acrylic monomeric units comprised in the polymer network comprised in the hydrogels of the present invention comprise an amount of between 2% and 15% by weight of silicon oxide acrylic monomeric units with respect to the total weight of acrylic monomeric units, this automatically generates an embodiment wherein the acrylic monomers comprised in the monomer mixture that is to be subjected to polymerisation comprise an amount of between 2% and 15% by weight of silicon oxide acrylic monomers with respect to the total weight of acrylic monomers. The monomer mixture refers to the group of compounds, such as monomers and initiator, that end up forming the polymer network.

Polymerisation reactions include thermal- or photo-polymerisation employing a radical initiator.

Examples of thermal polymerization initiator types are peroxides, hydroperoxides, azo-bis(alkyl- or cycloalkylnitriles), persulfates, percarbonates or mixtures thereof. Specific examples are benzoylperoxide, tert.-butyl peroxide, di-tert.-butyl-diperoxyphthalate, tert.-butyl hydroperoxide, azo-bis(isobutyronitrile) (AIBN), 1,1-azodiisobutyramidine, 1,1'-azo-bis (1-cyclohexanecarbonitrile), 2,2'-azo-bis(2,4-dimethylvaleronitrile). Preferably, the initiator is a thermal polymerization initiator, and more preferably it is AIBN.

The polymerization is carried out by subjecting the monomer mixture to an elevated temperature, for example to a temperature of from 30 to 100°C and preferably 40 to 80°C. The reaction time may vary within wide limits, but is conveniently, for example, from 1 to 48 hours. In a preferred embodiment, the polymerisation is carried out at 30-70°C for 6 to 18 h and then at 50-90°C for 18 to 30 h; such as 50°C for 12 h and then at 70°C for 24 h. It is advantageous to previously degas the components and solvents, if any, used in the polymerization reaction and to carry out said polymerization reaction under an inert atmosphere, for example under a nitrogen or argon atmosphere.

Examples of suitable solvents include, without limitation, tetrahydrofuran, tripropylene glycol methyl ether, dipropylene glycol methyl ether, ethylene glycol n-butyl ether, ketones (e.g., acetone, methyl ethyl ketone, etc.), diethylene glycol n-butyl ether, diethylene glycol methyl ether, ethylene glycol phenyl ether, propylene glycol methyl ether, propylene glycol methyl ether acetate, dipropylene glycol methyl ether acetate, propylene glycol n-propyl ether, dipropylene glycol n-propyl ether, tripropylene glycol n-butyl ether, propylene glycol n-butyl ether, dipropylene glycol n-butyl ether, tripropylene glycol n-butyl ether, propylene glycol phenyl ether dipropylene glycol dimetyl ether, polyethylene glycols, polypropylene glycols, ethyl acetate, butyl acetate, amyl acetate, methyl lactate, ethyl lactate, i-propyl lactate, methylene chloride, 2-butanol, 1-propanol, 2-propanol, menthol, cyclohexanol, cyclopentanol and exonorborneol, 2-pentanol, 3-pentanol, 2-hexanol, 3-hexanol, 3-methyl-2-butanol, 2-heptanol, 2-octanol, 2-nonanol, 2-decanol, 3-octanol, norborneol, tert-butanol, tert-amyl, alcohol, 2-methyl-2-pentanol, 2,3-dimethyl-2-butanol, 3-methyl-3-pentanol, 1-methylcyclohexanol, 2-methyl-2-hexanol, 3,7-dimethyl-3-octanol, 1-chloro-2-methyl-2-propanol, 2-methyl-2-heptanol, 2-methyl-2-octanol, 2-2-methyl-2-nonanol, 2-methyl-2-decanol, 3-methyl-3-hexanol, 3-methyl-3-heptanol, 4-methyl-4-heptanol, 3-methyl-3-octanol, 4-methyl-4-octanol, 3-methyl-3-nonanol, 4-methyl-4-nonanol, 3-methyl-3-octanol, 3-ethyl-3-hexanol, 3-methyl-3-heptanol, 4-ethyl-4-heptanol, 4-propyl-4-heptanol, 4-isopropyl-4-heptanol, 2,4-dimethyl-2-pentanol, 1-methylcyclopentanol, 1-ethylcyclopentanol, 1-ethylcyclopentanol, 3-hydroxy-3-methyl-1-butene, 4-hydroxy-4-methyl-1-cyclopentanol, 2-phenyl-2-propanol, 2-methoxy-2-methyl-2-propanol 2,3,4-trimethyl-3-pentanol, 3,7-dimethyl-3-octanol, 2-phenyl-2-butanol, 2-methyl-1-phenyl-2-propanol and 3-ethyl-3-pentanol, 1-ethoxy-2-propanol, 1-methyl-2-propanol, t-amyl alcohol, isopropanol, 1-methyl-2-pyrrolidone, N,N-dimethylpropionamide, dimethyl formamide, dimethyl acetamide, dimethyl propionamide, N-methyl pyrrolidinone, Dimethyl sulfoxide and mixtures thereof. When the monomer mixture comprises sulphonic acid anionic acrylic monomers, such as AMPSA, then the solvent preferably comprises dimethyl sulfoxide.

In a preferred embodiment, however, the polymerization is carried out in the absence of solvent. When the monomer mixture comprises sulphonic acid anionic acrylic monomers, such as AMPSA, then dimethyl sulfoxide is preferably used as sole solvent.

Examples of photoinitiators are benzoin methyl ether, 2,4,6-trimethylbenzoyldiphenylophosphine oxide, bis-(2,6-dichlorobenzoyl)-4-N-propylphenylphosphine oxide, bis-(2,6-dichlorobenzoyl)-4-N-butylphenylphosphine oxide, diethoxyacetophenone, 1-hydroxycyclohexyl phenyl ketone or Germane-based Norrish Type I photoinitiators. The polymerization can be triggered off by exposing a mixture comprising the monomer mixture and the photoinitiator to actinic radiation, for example light, in particular UV light.

Most preferably, the hydrogel precursor is prepared by a method as described in Pereira-da-Mota et al., Atorvastatin-Eluting Contact Lenses: Effects of Molecular Imprinting and Sterilization on Drug Loading and Release, Pharmaceutics, 2021 May; 13(5): 606.

In an embodiment, after the polymerisation and prior to subjecting the precursor hydrogel to step c), unreacted monomers are removed from the precursor hydrogel, such as by washing it with a solvent, such as water, until no monomer is detected in the solvent that was used for washing. Alternatively, unreacted monomers are removed from the precursor hydrogel by subjecting the precursor hydrogel to boiling, such as by immersing the precursor hydrogel in boiling water.

Additionally or alternatively, after the polymerisation and prior to subjecting the precursor hydrogel to step c), the precursor hydrogel is dried, such as by heating it above room temperature, such as at 50 °C.

Step b) in either case requires providing a gold nanoparticle precursor. A gold nanoparticle precursor herein refers to a composition comprising gold atoms in an oxidised state (typically (III) or (I) oxidation state) and in which said gold atoms do not form nanoparticles. The term "nanoparticle" in the present disclosure refers to a particle comprising at least one dimension of a size of 100 nm or less, such as of between 1 and 100 nm.

Non-limiting examples of gold nanoparticle precursors are $HAuCl_4$, $Au(NO_3)_3$, $Au(CH_3COO)_3$, $AuBr_3$, AuBr, and $Au(OH)_3$, as described in Shariq et al., Successful Synthesis of Gold Nanoparticles through Ultrasonic Spray Pyrolysis from a Gold(III) Nitrate Precursor and Their Interaction with a High Electron Beam, Chemistry Open, 2018, Volume 7, Issue 7, p. 533-542. Such precursors are readily commercially available from vendors such as Sigma-Aldrich, American Elements, Surepure Chemetals or ThermoFisher Scientific. In a preferred embodiment the gold nanoparticle precursor is selected from $HAuCl_4$ and $Au(CH_3COO)_3$, and more preferably the gold nanoparticle precursor is $HAuCl_4$.

Preferably, the gold nanoparticle precursor, such as $HAuCl_4$, is provided in the form of a solution. Providing the gold nanoparticle precursor in said form facilitates the penetration of the gold nanoparticle precursor throughout the polymer network comprised in the precursor hydrogel and thus maximises the efficiency of step c) in the methods of the present invention. The solution is preferably an aqueous solution, preferably a water solution. In an embodiment, the solution is a desalinated solution, such as distilled water. The term "desalinated" herein implies that the concentration of salts, such as of NaCl, is of 0.015 M or lower. Desalinating is preferred especially when the precursor hydrogel is an S-series precursor hydrogel.

In an embodiment, the concentration of the gold nanoparticle precursor, such as $HAuCl_4$, in said solution, preferably aqueous solution, is between 0.01 and 10 mM, more preferably between 0.1 and 1.0 mM, even more preferably between 0.3 and 0.7 mM. Additionally or alternatively, the gold nanoparticle precursor, such as $HAuCl_4$, solution, can be characterised by an acidic pH, such as by a pH of from 2 to 4, and in a preferred particular embodiment by a pH of 3.

Step c) in either case requires reducing the gold nanoparticle precursor to gold nanoparticles by bringing said precursor into contact (combining) with the precursor hydrogel. It has unexpectedly been found that the acrylic hydrogels of the present invention show affinity for the Au metallic ion in the gold nanoparticle precursor and are furthermore capable of reducing said ion within the acrylic network to generate gold nanoparticles.

Different methods known in the art can be employed for the combining of the precursor hydrogel and the gold nanoparticle precursor, such as immersing the precursor hydrogel in the gold nanoparticle precursor, drop-coating the gold nanoparticle precursor onto the precursor hydrogel, spraying the gold nanoparticle precursor onto the precursor hydrogel, or applying the gold nanoparticle precursor by a brush onto the precursor hydrogel. Any method may be used as long as gold nanoparticle precursor can be absorbed throughout the precursor hydrogel. Preferably, the combining of the precursor hydrogel and the gold nanoparticle precursor is by immersing the precursor hydrogel in the gold nanoparticle precursor, more preferably in a gold nanoparticle precursor solution as described above, even more preferably in an aqueous $HAuCl_4$ solution as described above.

The combining of the precursor hydrogel and the gold nanoparticle precursor is preferably performed for a period of time sufficient for the gold nanoparticle precursor to be absorbed throughout the precursor hydrogel. Preferably, combining or bringing into contact is by immersion as described above, and the precursor hydrogel is maintained immersed in the gold nanoparticle precursor for a period of at least 1 day, more preferably of at least 3 days. The combining of the precursor hydrogel and the gold nanoparticle precursor may be performed at any temperature which neither evaporates nor freezes either component, but can generally and preferably be carried out at room temperature (15 to 25 °C).

In a preferred particular embodiment, the combining of the precursor hydrogel and the gold nanoparticle precursor is performed by immersing the precursor hydrogel in an aqueous $HAuCl_4$ solution for a period of at least 3 days and at room temperature.

It has unexpectedly been found that when acrylic monomeric units comprised in the polymer network comprised in the precursor hydrogels comprise amine monofunctionalized acrylic monomeric units, preferably APMA, more preferably APMA in an amount of between 0.1 and 5%, particularly of between 0.4 and 1.0%, by weight with respect to the total weight of acrylic monomeric units, then the precursor hydrogels are capable of loading very high quantities of gold nanoparticle precursor.

The reduction in step c) of the methods of the invention is carried out in the dark. As used herein "in the dark" refers to intentionally limiting the amount of light to which the reduction is exposed, preferably to carrying out the reduction in the absence of light. More particularly, the term light refers to electromagnetic radiation in one or more, preferably in all, of the visible spectral range (380 to 770 nm), the ultraviolet spectral range (100 to 380 nm) and the infrared spectral range (770 nm to 1 mm). Alternatively, "in the dark" is measured by illuminance value. Preferably, the reduction is carried out at less than 20 lux, more preferably less than 10 lux, even more preferably less than 5 lux, yet more preferably less than 1 lux, such as from 0 lux to any of said values. Illuminance may be measured with a light meter, such as an ILT2400 Hand-Held Light Meter (International Light Technologies).

Method 1 precursor hydrogels of the present invention do not require the heat and pressure treatment in order to reduce the gold nanoparticle precursor to AuNPs. As can be appreciated in Figure 6 D-F, these hydrogels are capable of reducing the gold nanoparticle precursor to AuNPs by themselves. However, as shown in Figures 6 A-C, the heat and pressure treatment accelerates the formation of AuNPs. It was observed that approximately 30 days of immersion of the precursor hydrogel in an aqueous $HAuCl_4$ solution at room temperature and protected from light produced a hydrogel with a minimum in transmittance at the 500-900 nm range of the electromagnetic spectrum of a magnitude similar to that of a hydrogel for which the same immersion only lasted 3 days but was followed by 15 minutes of autoclaving at 121°C.

Nevertheless, it has been observed that the yield of the AuNPs formation is greater in the absence of the heat and pressure treatment. Therefore, in an embodiment, Method 1 of the invention comprises a step c) comprising combining the precursor hydrogel of step a) and the gold nanoparticle precursor of step b) in the absence of a heat and pressure treatment as described elsewhere herein, more particularly in the absence of autoclaving. In such cases, the combining of the precursor hydrogel and the gold nanoparticle precursor is performed as described above for a longer period of time (compared to when heat and pressure treatment is applied), such as for up to 200 days, in particular for up to 150 days. This slower but more efficient procedure also advantageously allows for a precise control of the percentage of gold reduction or of light blocking since the reaction can be stopped when the hydrogel is removed from the gold nanoparticle precursor.

It has also been found that precursor hydrogels wherein the acrylic monomeric units comprised in the polymer network do not comprise silicon oxide acrylic monomeric units, as well as precursor hydrogels wherein the polymer network comprises fluorinated or sulphonic acid monomers, are unable of reducing the gold nanoparticle precursor to AuNPs by themselves. However, most surprisingly, the reduction is enabled when a heat and pressure treatment is applied to the combination of the precursor hydrogel and the gold nanoparticle precursor.

The heat and pressure treatment may be applied immediately after combining the precursor hydrogel of step a) and the gold nanoparticle precursor of step b), but is more preferably applied after allowing sufficient time for the gold nanoparticle precursor to be absorbed throughout the precursor hydrogel. Therefore, in an embodiment, the combining of the precursor hydrogel of step a) and the gold nanoparticle precursor of step b) is performed in the absence of the heat and pressure treatment for a period of time, such as for at least 1 day, such as for a period of 3 days, before

subjecting the combination to the heat and pressure treatment. Said prior combining is preferably by immersing the precursor hydrogel in an aqueous $HAuCl_4$ solution at room temperature as was described above.

As used herein, "heat and pressure treatment" refers to a treatment which subjects the combination of the precursor hydrogel of step a) and the gold nanoparticle precursor of step b) to a temperature of at least 60 °C, such as of between 60 and 180 °C, and to a pressure above atmospheric pressure (e.g. 101.3 kPa), such as of between greater than 101.3 kPa and 300 kPa.

Preferably, said temperature is of between 90 and 150 °C, more preferably of between 120 to 134 °C, and in a preferred and particular embodiment said temperature is 121 °C.

Preferably, said pressure is of between 150 and 250 kPa, more preferably of between 200 and 210 kPa, and in a preferred and particular embodiment said pressure is 204.7 kPa. Alternatively, the pressure can be expressed with respect to the increment in pressure over atmospheric pressure. Thus, the pressure may be of between 50 and 150 kPa, preferably of between 100 and 110 kPa, and in a preferred and particular embodiment of 103.4 kPa, above atmospheric pressure.

The heat and pressure treatment is preferably applied for a period of between 3 minutes and 2 hours, more preferably of between 10 and 30 minutes, and in a preferred and particular embodiment said period is of 15 to 20 minutes.

In a preferred embodiment, these temperatures, pressures and times are combined.

In a very preferred embodiment, said heat and pressure treatment comprises subjecting the combination of the precursor hydrogel of step a) and the gold nanoparticle precursor of step b) to a gas, preferably to water vapour (*aka* wet steam), more preferably to saturated steam, at a temperature and pressure and during a period of time as described above. "Saturated steam" refers to water vapour in a state of equilibrium between condensation and evaporation. In addition to enabling the rapid formation of AuNPs, this heat and pressure treatment advantageously and additionally serves to sterilise the hydrogel to standards required for instance in the ophthalmology industry. When this heat and pressure treatment is carried out in an autoclave, the process is referred to as autoclaving. Most preferably, the heat and pressure treatment refers to autoclaving. Autoclaving can be performed as indicated in ISO standard 17665-1:2006(en).

Once the heat and pressure treatment is completed, i.e. once the reduction of the gold nanoparticle precursor to gold AuNPs throughout the precursor hydrogel is effected, hydrogels according to the present invention are obtained.

The heat and pressure treatment may be performed more than one time, with optional immersing of the hydrogel in a different/fresh gold nanoparticle precursor solution as described above in between each heat and pressure treatment. However, it has been found that a single heat and pressure treatment is sufficient to yield hydrogels according to the present invention.

What is common to every method of the present invention is that the reduction of step c) proceeds in the absence of any compound other than the precursor hydrogel of step a) and the gold nanoparticle precursor of step b), more specifically in the absence of reagents used in the prior art for such purpose, such as, sodium borohydride, $H_2O_2$, $AgNO_3$, thiols, sulphides such as $Na_2S$ or sulfur oxides such as dithionite, carboxylic acids such as citric, acetic, oxalic, adipic, ascorbic, benzoic or oleic acid or their salts, sulfonic acids such as 4-(2-hydroxyethyl)-1-piperazine propanesulfonic acid or their salts, hydroquinone, trihydroxybenzene, acetanilides, dimethylformamide (DMF), acetonitrile, 4-aminoantipyrine, carboxymethyl cellulose (CMC); a further polymer (in addition to the polymer network comprised in the hydrogels of the present invention); surfactants such as Triton X -100 (*aka* Polyethylene glycol p-(1,1,3,3-tetramethylbutyl)-phenyl ether), cetyltrimethylammonium chloride (CTAC), cetyltrimethylammonium bromide (CTAB), didecyldimethylammonium bromide (DDAB), cetylpyridinium chloride (CPC), tetraoctylammonium bromide (TOAB), sodium dodecyl sulfate (SDS), sodium bis(2-ethylhexyl)-sulfosuccinate, n-decanol, lauryl sulfobetaine, oleylamine, lecithin, phospholipids; amino acids such as Ser, Thr, Tyr, Trp, Lys; proteins such as serum abumin; plant extracts, e.g. from Plectranthus saccatus, Malva Parviflora, Plumbago zeylanica, Capsicum annuum var. grossum, Indigofera tinctoria, Lantana camara berry, Musa paradisiaca peel, Artemisia dracunculus, Cucurbita maxima, Lantana camara Linn root, Cacumen Platycladi, Ampelopsis grossedentata, Couroupita guianensis Aubl., Red beetroot peel, Alchemilla mollis, Carica papaya and Catharanthus roseus, or specific compound types found therein such as polyphenols, alkaloids, flavonoids, steroids, glycosides, carbohydrates, saponins, tannins, Bael gum; buffers comprising 1,4-piperazinediethanesulfonic acid (PIPES), 3-{[1,3-dihydroxy-2-(hydroxymethyl)propan-2-yl]amino}-2-hydroxypropane-1-sulfonic acid (TAPSO), 3-{[1,3-dihydroxy-2-(hydroxymethyl)propan-2-yl]amino}propane-1-sulfonic acid (TAPS), 2-[bis(2-hydroxy)ethylamino]acetic acid (bicine), 2-{[1,3-dihydroxy-2-(hydroxy-methyl)propan-2-yl]amino}ethanesulfonic acid (TES), tris(2-hydroxyethyl)amine (TEA), 2-[bis(2-hydroxyethyl)amino]-2-(hydroxymethyl)propane-1,3-diol (BTM), 2,20-(1,3-Propanediyldiimino)bis[2-(hydroxymethyl)-1,3-propanediol], 3-(N-morpholino)propanesulfonic acid (MOPS), 2-[4-(2-hydroxyethyl)piperazin-1-yl]ethanesulfonic acid (HEPES); or microorganisms; and in the absence of added AuNP seeds.

This is highly advantageous in that the use of other reagents can interfere with the polymer network comprised in the precursor hydrogel, and even damage it such as when strong reducing agents are employed. Furthermore, no additional washing step is required for removing the other reagent from the obtained hydrogel after the reduction. If not removed, or not entirely removed, the other agent might compromise the intended use of the hydrogel, such as ocular

safety when ophthalmologic uses are intended for the hydrogel.

Preferably, the reduction of step c) proceeds in the absence of a reducing agent (other than the precursor hydrogel) such as sodium borohydride, or citrate or a salt thereof; and/or of a surfactant such as CTAB.

The marked anisotropic nature of the AuNPs comprised in the hydrogels of the present invention enables the use of said hydrogels in a wide range of industrial applications.

A further aspect of the invention relates to the hydrogels of the invention for use in medicine, preferably in ophthalmology.

Thus, in an embodiment, the present invention relates to a hydrogel of the present invention in the form of a contact lens. Alternatively, in a further aspect, the present invention relates to a contact lens comprising a hydrogel according to the present invention. In another aspect, the present invention relates to a method for preparing said contact lens.

In an embodiment, the contact lens is a soft contact lens. In the context of the present invention, a soft contact lens is a contact lens having an elastic modulus (i.e., Young's modulus) of less than 2.5 MPa.

Different methods for preparing the contact lens may be employed. For instance, the polymerization reaction of step a) of the methods of the present invention can be carried out in a mold with a contact lens shape, such that the precursor hydrogel is already provided in the form of a contact lens. If the polymerization reaction has not been carried out in a mold providing the hydrogel with a contact lens shape, the contact lens can be prepared by means of lathe-cutting the precursor hydrogel or the hydrogel of the invention, or by means of molding the same, particularly by means of centrifugal molding or by means of cast molding, or by means of combinations of these techniques.

Further aspects of the invention relate to the hydrogels of the invention, preferably in the form of a contact lens, for use in the treatment of:

- infection, such as infection by *Staphylococcus epidermidis, Citrobacter freundii, Staphylococcus aureus, Bacillus subtilis, Proteus vulgaris, Pseudomonas aeruginosa,* or *Escherichia coli.*
- eye tumours of the anterior segment.
- posterior capsule opacification associated to intraocular lenses.
- ocular cystinosis.
- color blindness.

Another important aspect refers to the hydrogels of the invention, preferably in the form of a contact lens, for use in *in vivo* biological imaging.

The present invention also embraces methods of treatment of any one of the above medical conditions in a subject in need thereof, comprising administering a hydrogel according to the present invention to said subject.

The present invention also embraces the use of a hydrogel according to the present invention in the manufacture of a medicament for the treatment of any one of the above medical conditions.

Additionally, another aspect of the invention relates to the use of the hydrogels of the invention in *ex vivo* biological imaging and biosensing.

Non-medical uses of the hydrogels of the invention can be as catalysts or in molecular detection by Surface-enhanced Raman spectroscopy (SERS).

The invention is further illustrated, but not limited by, the following examples.

## Examples

*Materials*

Hydrogen tetrachloroaurate(III) ($HAuCl_4$), ethylene glycol dimethacrylate (EGDMA), dichlorodimethylsilane, benzyl methacrylate (BzMA), ethylene glycol phenyl ether methacrylate (EGPEM), methacrylic acid (MAA), 2-acrylamido-2-methylpropane sulfonic acid (AMPSA) and 2,2'-azobis(2-methylpropionitrile) (AIBN) were from Sigma Aldrich (St Louis, MO, USA). 2-Hydroxyethyl methacrylate (HEMA) was from Merck KGaA (Darmstadt, Germany). N-(3-aminopropyl) methacrylamide hydrochloride (APMA) was from PolySciences Inc. (Warrington, Pennsylvania, USA). Monomethacryloxypropyl-sym-polydimethylsiloxane hydroxypropyl terminated (MCS-MC12) was from Gelest, Inc (Morrisville, PA, USA). Distilled water was used for all experiments. All other reagents were analytical grade. Commercially available CLs were Esencia® 50 from Eurolent Servicios Opticos S.L. (Madrid, Spain), Unisil from Contamac UK, Biofinity Energys™ from Cooper Vision (Hamble, UK), Acuvue® Oasys with HydraLuxe™ from Johnson & Johnson Vision Care Company (Limerick, Ireland), and 1-Day Acuvue® Moist® with Lacreon® from Johnson & Johnson Vision Care Company (Limerick, Ireland). Commercial CLs were acquired from local optician's shops and were extensively washed with water in individual baskets for 24 h and dried at 50°C for 12 h before testing.

*Example 1 - Precursor hydrogel synthesis*

Various precursor hydrogel batches differing in monomeric unit composition (see Table 1 for %weight composition and structures of monomers below Table 1) were prepared as previously described [A.F. Pereira-da-Mota, M. Vivero-Lopez, A. Topete, A. P. Serro, A. Concheiro, C. Alvarez-Lorenzo. Atorvastatin-Eluting Contact Lenses: Effects of Molecular Imprinting and Sterilization on Drug Loading and Release. Pharmaceutics 2021, 13(5):606]. Briefly, monomer solutions comprising AIBN as initiator and DMSO (0.5 mL; not used for AE series) were injected in square side glass moulds (10x10 cm) prepared with a silicone frame of 0.2 mm thickness, and the polymerization was carried out at 50 °C for 12 h and at 70 °C for another 24 h. Then, the obtained precursor hydrogels were boiled, cut as 10 mm discs or formed into contact lenses (CLs), and extensively washed until no monomer leakage was detected. The discs were dried at 50°C for 24 h.

| ID | HEMA | BzMA | MCS-MC12 | MAA | AMPS A | EGPEM | APMA | EGDMA |
|---|---|---|---|---|---|---|---|---|
| H1 | 99.8492 (4g) | 0.0000 | 0.0000 | 0.0000 | 0.0000 | 0.0000 | 0.0000 | 0.1508 |
| H2 | 98.9991 (4g) | 0.0000 | 0.0000 | 0.8514 | 0.0000 | 0.0000 | 0.0000 | 0.1495 |
| H3 | 98.1610 (4g) | 0.0000 | 0.0000 | 1.6908 | 0.0000 | 0.0000 | 0.0000 | 0.1482 |
| H4 | 99.8472 (4g) | 0.0000 | 0.0000 | 0.0000 | 0.0021 | 0.0000 | 0.0000 | 0.1508 |
| H5 | 99.8451 (4g) | 0.0000 | 0.0000 | 0.0000 | 0.0041 | 0.0000 | 0.0000 | 0.1508 |
| B1 | 96.4543 (4g) | 3.4000 | 0.0000 | 0.0000 | 0.0000 | 0.0000 | 0.0000 | 0.1456 |
| B2 | 95.6608 (4g) | 3.3720 | 0.0000 | 0.8227 | 0.0000 | 0.0000 | 0.0000 | 0.1444 |
| B3 | 94.8780 (4g) | 3.3444 | 0.0000 | 1.6343 | 0.0000 | 0.0000 | 0.0000 | 0.1433 |
| B4 | 96.4524 (4g) | 3.3999 | 0.0000 | 0.0000 | 0.0020 | 0.0000 | 0.0000 | 0.1456 |
| B5 | 96.4505 (4g) | 3.3999 | 0.0000 | 0.0000 | 0.0040 | 0.0000 | 0.0000 | 0.1456 |
| S1 | 87.2365 (3.5g) | 0.0000 | 12.4624 | 0.0000 | 0.0000 | 0.0000 | 0.0000 | 0.3011 |
| S2 | 86.4949 (3.5g) | 0.0000 | 12.3564 | 0.8501 | 0.0000 | 0.0000 | 0.0000 | 0.2985 |
| S3 | 85.7637 (3.5g) | 0.0000 | 12.2520 | 1.6883 | 0.0000 | 0.0000 | 0.0000 | 0.2960 |
| S4 | 87.2347 (3.5g) | 0.0000 | 12.4621 | 0.0000 | 0.0021 | 0.0000 | 0.0000 | 0.3011 |
| S5 | 87.2329 (3.5g) | 0.0000 | 12.4618 | 0.0000 | 0.0041 | 0.0000 | 0.0000 | 0.3011 |
| AE 1 | 96.0129 (3g) | 0.0000 | 0.0000 | 0.0000 | 0.0000 | 3.6005 | 0.0000 | 0.3866 |
| AE2 | 95.3583 (3g) | 0.0000 | 0.0000 | 0.0000 | 0.0000 | 3.5759 | 0.6818 | 0.3840 |
| AE3 | 98.8947 (3g) | - | - | - | - | - | 0.7071 | 0.3982 |

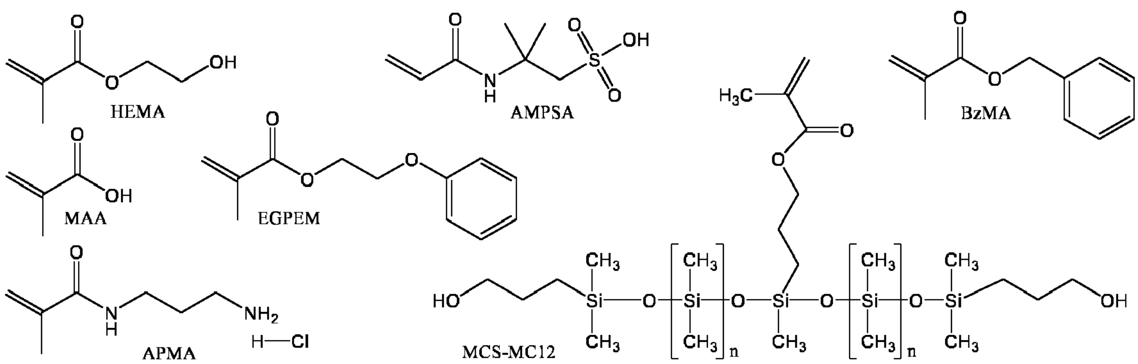

HEMA, AMPSA, BzMA, MAA, EGPEM, APMA, MCS-MC12

Additionally, the commercially available CLs described above were also employed as precursor hydrogels.

Lastly, a second S-series of hydrogels was prepared. These hydrogels were labelled Sb1, Sb2 and Sb3 and their monomeric composition was identical to that of hydrogels S 1, S2 and S3, the only difference being that Sb hydrogels were prepared in the absence of DMSO in order to discard any effect DMSO could be having on experimental results.

*Example 2 - Preparation of hydrogels of the invention*

All glassware was carefully washed with HCl 10 mM and then rinsed with distilled water to remove potentially interfering ions. Dried precursor hydrogel discs or CLs were weighed and individually placed in freshly prepared $HAuCl_4$ solution in water (0.327 mM, 2 mL). Six replicates of each disc/CL were evaluated in each condition. The vials were capped and stored for 72 h at room temperature protected from light. Light transmission of the discs/CLs was measured. After the measurement, the discs/CLs were immediately returned to the same $HAuCl_4$ solution, and the vials were sealed and autoclaved at 121 °C for 15 min. Then, the vials were left to equilibrate at room temperature for 4 h, and the light transmission of the discs/CLs was recorded again. Commercial CLs were washed in water for 24 h to remove components from the maintenance liquid that may interfere in the reduction process. Transmittances of the commercial CLs were recorded before and after soaking in $HAuCl_4$ solution (0.327 mM, 2 mL) for 72 h, as well. Some replicates underwent steam heat sterilization (autoclave, 121 °C, 20 min) and the other replicates were stored until day 7 at room temperature, and the transmittance was recorded again.

Sb series hydrogels were treated in two manners: as for S1 hydrogels and also by prolonging the immersion in $HAuCl_4$ solution up to 150 days.

*Example 3 - Gold nanoparticle precursor sorption and light transmittance of hydrogels Gold nanoparticle precursor sorption measurement*

In order to determine the amount of gold nanoparticle precursor sorbed by the disc/CL precursor hydrogels, the UV-Vis spectra of gold precursor solutions were recorded in the 190-800 nm range (Agilent 8453, Germany) after the 72 h storage period and before autoclaving, and then again after autoclaving.

*Light transmittance measurement*

Light transmission of the discs and CLs was evaluated in the 200-900 nm range (1 nm step) using an Agilent Cary 60 UV-Vis spectrophotometer (Santa Clara, CA, USA). The discs were removed from the solution, their surface was carefully wiped with sorbent paper, and the discs were directly placed in a support for solid samples. The measurements were carried out in air after zeroing the transmittance at 900 nm. Transmittance spectra were also recorded in the 200-2000 nm interval (1 nm; 1000 nm/min), after baseline correction in a V-770 UV-Visible/NIR spectrophotometer fitted with VTA-752 sample accessory (Jasco, Tokyo, Japan).

*Results*

Hydrogels prepared from precursor hydrogel compositions reported in Table 1 had transmittances in the 500-800 nm wavelength region above 90% (ideal for their use as CLs), which allowed for easy monitoring of AuNP formation through visual changes in color and UV-vis spectrophotometric recording of transmittance. Transmittances after autoclaving as well as the amount of gold ions sorbed by the hydrogels are shown in Figure 2. The amounts of gold precursor sorbed by the hydrogels at room temperature are shown on the left bars for each hydrogel in Figure 2E. $HAuCl_4$ (0.327 mM) solution in water (pH 3) showed a yellowish color with a peak in absorbance at 287 nm well in the range of the UV-Vis spectrophotometer measurements. After 72 h immersion, the absorbance of the medium decreased considerably for all hydrogels except those bearing AMPSA (H4, H5, S4, S5, B4 and B5), for which the decrease was moderate. Remarkably, AE2 and AE3 discs sorbed almost all gold salt in solution, acquiring yellowish color. Steam heat sterilization of control $HAuCl_4$ (0.327 mM) aqueous solution caused a minor decrease in the absorbance at 287 nm and nonadditional peaks appeared, which indicated that the autoclaving itself did not induce gold reduction neither AuNPs formation in the gold nanoparticle precursor solution in the absence of the precursor hydrogel. The small decrease in absorbance due to autoclaving was taken into account when calculating the amount of gold entrapped by the hydrogels. In the presence of discs the absorbance of the medium at 287 nm diminished more than 50%, revealing the high capability of the hydrogels to uptake gold ions (Figure 2E, right bars). Only the AE2 and AE3 hydrogels that had sorbed almost all gold precursor in solution at room temperature had expelled some amount of gold precursor after autoclaving.

Regarding the discs, all compositions depicted in Table 1 underwent a remarkable change in color after steam heat sterilization. The recording of the light transmission of the discs after sterilization evidenced a decrease in transmittance in the 532-652 nm region. Regarding hydrogels without silicone oxide monomer, the presence of low contents in MAA favored the decrease in transmittance in the 558 nm region (hydrogels H2 and B2). In the case of B hydrogels, those prepared with AMPSA had intermediate behavior between non-functionalized and MAA functionalized ones (Figure 2 C). Silicone oxide hydrogels S 1 (without MAA or AMPSA) underwent the strongest decrease in transmittance, followed by S2 and S3 prepared with MAA.

Turning to commercial hydrogels, CLs made of HEMA hydrogel and minor contents in NVP and MMA (Esencia®

50) performed similarly to H discs series (Figure 3 A). Silicone hydrogel Unisil CLs, which contains a non-disclosed mixture of HEMA, NVP, TRIS and SIGMA, sorbed a large amount of gold in solution and transformed it into AuNPs becoming tinted before autoclaving, as observed for silicone hydrogels S1, S2 and S3 (Figure 3 A). A similar behavior is observed for Pure Vision CLs, which are predominantly based on Si-containing vinylcarbamate units (Figure 3 F). Acuvue® Oasys with HydraLuxe™, which is made of senofilcon A and contains 9.7-12.8% Si in the outermost region of the CL, performed similarly to Unisil CLs and triggered an intense formation of AuNPs after 3 days in the gold precursor solution (Figure 3 C). The CLs become more intensely colored after autoclaving. Surprisingly, Biofinity Energys™ did not induce the reduction of gold (Figure 3 B) despite being a silicone hydrogel too. These CLs are made of comfilcon A, the only CL with a significant content in fluorine. Biofinity Energys™ CLs required steam heat sterilization for the triggering of AuNP formation. Another unexpected behavior was found for 1-Day Acuvue® Moist® with Lacreon® which is made of etafilcon A, i.e. a copolymer of HEMA and MAA cross-linked with EGDMA and 1,1,1-trimethylol propane trimethacrylate, as well as polyvinyl pyrrolidone (PVP), which has previously been demonstrated able to induce AuNP formation by itself. These CLs reduced gold at room temperature (Figure 3 D) and become reddish after 3 days storage before autoclaving. However, reduction at room temperature yielded hydrogels with a transmittance minimum in the 505-525 nm range, irrespective of whether they were immersed in the $HAuCl_4$ solution for 3 or 7 days. Unexpectedly, when autoclaved, the minimum absorbance of these hydrogels upshifted to the 550-570 nm range.

As regards Sb series hydrogels, this new batch showed homogeneous red-purple staining in few hours. The color was faint in the first three days of incubation. Half replicates (n=3) were autoclaved after 3 days incubation in $HAuCl_4$ solution (0.327 mM, 2 mL) (Figure 6 A, B, C) to serve as positive controls of the maximum reducing capability. As observed above for other silicone hydrogels, steam heat sterilization caused an immediate decrease in transmittance in the 532-652 nm region, and prolonged incubation in the same precursor solution after autoclaving only caused minor further decrease in the transmittance. This suggests that a maximum in gold conversion was reached during autoclaving, which did not increase during extended storage in $HAuCl_4$ solution. Unexpectedly, a shift in the transmittance peak from 550 to 570 nm (redder wavelengths) was observed as the content in MAA in the silicone hydrogels increased (Figure 6 C), which could be attributable to the formation of larger particles. Overall, autoclaving was shown to be a simple and very efficient method to produce anisotropic AuNPs inside CL using much lower concentrations of gold ions than when using inorganic catalysts, which furthermore only generate spherical particles. After autoclaving, remarkably low transmittances in the 550-570 nm interval were achieved: 38% transmittance for silicone hydrogels without MAA and 43-50% transmittance for silicone hydrogels with MAA, while still allowing vision through them.

Replicates that did not undergo autoclaving (n=3) but remained stored in $HAuCl_4$ solution evidenced a progressive decrease in the transmittance in the 500-700 nm region (Figure 6 D, E, F), which was shown as a remarkable increase in the red-purple staining. It took approx. 30 days of incubation for the hydrogels to reach the same decay in transmittance as shown by the autoclaved hydrogels (121 °C, 15 min). The minimum in transmittance that was recorded after 60 days of incubation (20%, 30% and 35% for S1b, S2b, and S3b, respectively) was below of that recorded after autoclaving. In this regard, the slow drop in absorbance at 287 nm of the external gold solution suggested more efficient reduction of gold and growth of AuNPs inside the hydrogel at 20 °C than during autoclaving. The hydrogels incubated at room temperature required more time but less gold ions to block the light to the same extent as autoclaved hydrogels. After 3 months storage, S1b, S2b, and S3b had sorbed ca. 100% gold ions from the medium and the transmittance at 550, 564 and 570 nm was of 12.8, 27.6, and 30.8 % respectively (Figure 6 D, E, F). The slow AuNPs formation at 20°C allowed for a precise control of the percentage of gold reduction or of light blocking since the reaction stopped when the hydrogels were transferred to water. Relevantly for the practical use as CLs, the strong blocking of the transmittance attained after 5 months storage still allowed seeing through the hydrogels with high sharpness.

To gain a further insight into the light absorption features of the S1b, S2b and S3b hydrogel discs, transmittance spectra were recorded in the 200-2000 nm range. The spectra (Figure 7) confirmed the high light transmission of the hydrogels before any treatment and evidenced a decrease in transmittance from 550 nm toward the red region that expanded in the near-infrared region.

*Example 4 - Photothermal responsiveness*

Responsiveness of the hydrogels of the invention to light was evaluated using three laser pointers covering a wide range of wavelengths: a bullet 100 mW green laser pointer 532 nm (beam diameter 2.0 mm), a bullet 100 mW red laser pointer 652 nm (beam diameter 2.0 mm), and a IR1 laser pointer 808 nm 100 mW (beam diameter 3.0 mm) (Biglasers.com, Monroe, Ny, USA). Individual hydrogel wet discs (i.e. not subjected to drying after their preparation) were placed on a lux light meter sensor (model SBS-LM-400C; Steinberg Systems, Zielona Gora, Poland) and irradiated with the laser of choice at a distance of 19 cm. The changes in temperature were recorded with a FLIR model E75 thermographic camera (Teledyne FLIR LLC, Oregon), taking thermal images at a focal distance of 19 cm at the beginning of the process and after 10 seconds laser exposure. Non-treated discs (without AuNPs) did not change either illuminance or temperature after 10 s irradiation with any laser pointer in good agreement with their transmittance spectra. Also, the

power meter sensor did not change its temperature after 10 s irradiation with any of the lasers. Discs that were prepared by soaking in $HAuCl_4$ solution (0.327 mM, 2 mL) for 72 h, subsequent steam heat sterilization (autoclave, 121 °C, 20 min), and then storage in water for 6 months increased 10 to 25 °C their temperature when irradiated with the green laser for 10 s. These discs blocked 20-70 % of light radiation depending on the hydrogel composition (Figure 8). As expected from Figure 2, S1 hydrogels incubated for 3 days in $HAuCl_4$ and then steam heat sterilized that were the most efficient ones in terms of gold reductant capability, showed the greatest light blockage capability. These same discs also showed photothermal performance when irradiated with the NIR 808 nm laser, although with smaller increases in temperature ranging from 7 to 16 °C and light blockage of 30-50% (Figure 8). S1b, S2b and S3b hydrogel discs that were incubated in $HAuCl_4$ (0.327 mM, 2 mL) solution prepared in water and kept at 20°C for 143 days were the most efficient ones in terms of both photothermal effect and radiation blockage (Figure 9). When the hydrogels were irradiated with the 532 nm laser the temperature increased 45-65 °C in 10 s and decreased the illumination up to 37.5%. Exposition to red laser caused an increase in temperature of 28-29 °C and the illumination decreased 55-65%, while exposition to near-infrared laser increased the temperature 15-25 °C and the illumination decreased 45-65%. These results evidenced the suitability of the polymer-triggered gold reduction to prepare hydrogels with the capability to absorb radiation in the near-infrared physiological window, which opens a wide range of therapeutic applications.

*Example 5 - Gold nanoparticle characterization: X-ray spectroscopy, Scanning electron microscopy, Size and Circularity*

*X-ray spectroscopy*

Hydrogels were washed for one week with water and freeze-dried before X-ray analysis. Crystalline powder X-ray diffraction (XRPD) data were obtained using a Bruker D8 Advance diffractometer (40 kV, 40 mA, theta/theta) equipped with an X-ray tube of sealed Cu (CuK$\alpha$1; $\lambda$= 1.5406 Å) and a LYNXEYE EQUIST-type detector. The diffractograms were obtained in the angular range 3-70° with a step of 0.02° and a time per step of 2s, and the diffractograms obtained were analyzed using HighScore Plus, version 3.0d software.

X-ray spectra of the hydrogels evidenced the amorphous state of the polymer network, with broad bands in the 20-30 °2$\theta$ range, and three crystalline peaks at 38.078 (intense), 44.256 and 64.376 °2$\theta$ (Figure 4). These peaks are typical of Au face center cubic lattice nanocrystals and thus confirmed that pure AuNPs were formed inside the cross-linked networks.

*Scanning electron microscopy (SEM)*

Images were recorded in a ZEISS GEMINI-500 (BSD4 detector) with EDX (UltimMax-170; Oxford, 127ev) at various magnifications (see Figure 1).

SEM images evidenced that the AuNPs were highly anisotropic showing heterogeneous sizes and shapes, with a predominance of truncate triangle and hexagonal plates (Figure 1 A-G).

The developed hydrogels facilitated by themselves, or with the aid of autoclaving, the formation of anisotropic AuNPs. Autoclaving favored the process, providing in few minutes large particles, which many be particularly useful for in the red light-near infrared applications. Even for Biofinity Energys™ silicone CLs which did not reduce gold ions at room temperature even after weeks of storage, steam heat sterilization of gold-ion soaked CLs triggered the formation of anisotropic AuNPs, although at much lesser extent than for the other hydrogel compositions tested.

*Size & Circularity*

1-ImageJ 1.53k software, preferably version 1.52p, from Wayne Rasband and contributors is in the public domain and available from National Institutes of Health, USA, at http://imagej.nih.gov/ij

Original SEM images (tiff files) obtained as described above were used to set the scale, as follows:
Draw line over the scale bar and select Analyze → Set Scale; In Set Scale window enter the value of the scale bar depicted in the original tiff file into the 'Known Distance' box; Then, Change the 'Unit of Measurement' box to $\mu$m, and check 'Global'. The obtained scale value was annotated for each SEM image.

2- Each SEM image was opened using the Snip & Sketch Windows 10 tool. The portion where the particles were depicted was selected, and the file saved as JPG file.

3.-In the ImageJ software proceed as follows: File Open (the JPG file was chosen); Image

- Type - 8 byte; Analyze - Set scale (For example, for 50000x with 400 nm scale bar, the scale was 0.1825 pixel/nm);

Image - Adjust - Threshold: Dark background; Analyze

- Particles: Pixel units, Circularity 0.00-1.00, Show Overlay, Display results. A table is automatically generated for all particles measured, containing number of particle, area, perimeter, and Feret diameter.

4.- The Table was transferred to Excel and the mean values and standard deviations of all parameters calculated.

The size of the particles is herein defined as the Feret diameter, the mean size as the mean Feret diameter.

Circularity was calculated for each individual particle applying the formula $C = 4 \pi A / P^2$, where A is the two-dimensional projection area of the form and P is the two-dimensional projection perimeter of the form. The area (A) and perimeter (P) values were directly taken from the values provided by ImageJ software as described above.

The circularity of all particles analyzed in each image was expressed as mean value. Exemplary mean sizes of the gold nanoparticles in the hydrogels of the invention were as follows: H1: 154.3 nm; B1: 289.8 nm; S1: 198.2 nm; BE: 339.3 nm; Pure Vision: 115.2 nm.

Exemplary mean circularities of the gold nanoparticles in the hydrogels of the invention were as follows: H1: 0.7450; B1: 0.6537; S1: 0.7037; BE: 0.8308; Pure Vision: 0.766.

*Example 6 - Content in water*

One concern associated to the loading of AuNPs inside CLs during polymerization or using strong inorganic reductants is related to the significant decrease in water content of the hydrogels, which may compromise their biomedical applications. The values recorded for H, S, B and AE hydrogels were in good agreement with those previously reported for related hydrogels. Also, the content in water obtained for the commercial CLs agreed well with the nominal content in water of Esencia® 50, Unisil, Biofinity® Energys™, Acuvue® Oasys and Acuvue® Moist, which are 50%, 62%, 40%, 38%, and 58%, respectively. Results are shown in Figure 5.

*Example 7 - AuNP leakage from hydrogels*

Discs/CLs comprising AuNPs were rinsed with water and transferred to Eppendorf tubes containing 4 mL distilled water, or NaCl 0.9% medium (isotonic with physiological fluids). After one week storage, the medium did not show absorbance in the 200-800 nm range which meant that the AuNPs did not leach from the hydrogels. Also, the transmittance pattern of the stored discs with AuNPs formed inside did not change after soaking in distilled water or NaCl 0.9% medium for one week and remained the same six months later.

**Claims**

1. Hydrogel comprising:

   - a polymer network, wherein the polymer network:

     ◦ comprises acrylic monomeric units in an amount of at least 90% by weight with respect to the total weight of the polymer network; and
     ◦ is cross-linked;

   - gold nanoparticles,

     wherein the hydrogel exhibits a light transmittance minimum at the 532-652 nm range within the range 500-900 nm of the electromagnetic spectrum; and
     wherein the hydrogel exhibits a temperature increase of at least 7°C following irradiation with a 100 mW laser at a wavelength of 808 nm during 10 s.

2. Hydrogel according to claim 1, wherein the gold nanoparticles exhibit a mean circularity value of less than 0.85.

3. Hydrogel according to any one of the preceding claims, wherein the size of the shortest dimension of the hydrogel is greater than 1 μm, preferably greater than 10 μm, even more preferably greater than 50 μm.

4. Hydrogel according to any one of the preceding claims, wherein the acrylic monomeric units comprised in the polymer network comprise an amount of at least 90% by weight of monofunctionalized acrylic monomeric units, and an amount of between 0.01% and 10% by weight of bifunctionalized acrylic monomeric units; with respect to the total

weight of acrylic monomeric units.

**5.** Hydrogel according to any one of the preceding claims, wherein the acrylic monomeric units comprised in the polymer network comprise an amount of between 0.001% and 5%, preferably of between 0.002% and 2%; by weight of anionic acrylic monomeric units with respect to the total weight of acrylic monomeric units.

**6.** Hydrogel according to any one of the preceding claims, wherein the acrylic monomeric units comprised in the polymer network comprise an amount of between 2% and 15%, preferably of between 3% and 5%; by weight of aromatic acrylic monomeric units with respect to the total weight of acrylic monomeric units

**7.** Hydrogel according to any one of the preceding claims, wherein the acrylic monomeric units comprised in the polymer network comprise an amount of between 2% and 90%, preferably of between 2% and 15%; by weight of silicon oxide acrylic monomeric units with respect to the total weight of acrylic monomeric units.

**8.** Hydrogel according to any one of the preceding claims, wherein the polymer network does not comprise any monomeric unit different to acrylic monomeric units.

**9.** Contact lens comprising a hydrogel as defined in any one of the preceding claims.

**10.** Method for preparing a hydrogel, comprising the steps of:

a) Providing a precursor hydrogel comprising a polymer network, wherein the polymer network:

◦ comprises acrylic monomeric units in an amount of at least 90% by weight with respect to the total weight of the polymer network, wherein said acrylic monomeric units comprise silicon oxide acrylic monomeric units;
◦ does not comprise sulphonic acid monomeric units;
◦ comprises, at most, 1% by weight fluorinated monomeric units with respect to the total weight of the polymer network; and
◦ is cross-linked;

b) Providing a gold nanoparticle precursor;
c) Reducing the gold nanoparticle precursor to gold nanoparticles, wherein the reduction comprises combining the precursor hydrogel of step a) and the gold nanoparticle precursor of step b) and is carried out in the dark, in the absence of a reducing agent and in the absence of a surfactant.

**11.** Method for preparing a hydrogel, comprising the steps of:

a) Providing a precursor hydrogel comprising a polymer network, wherein the polymer network:

◦ comprises acrylic monomeric units in an amount of at least 90% by weight with respect to the total weight of the polymer network; and
◦ is cross-linked;

b) Providing a gold nanoparticle precursor;
c) Reducing the gold nanoparticle precursor to gold nanoparticles, wherein the reduction:

- comprises combining the precursor hydrogel of step a) and the gold nanoparticle precursor of step b), and subjecting said combination to a heat and pressure treatment;
- is carried out in the dark, in the absence of a reducing agent and in the absence of a surfactant.

**12.** Method according to claim 11, wherein the heat and pressure treatment is autoclaving at a temperature of between 120 to 134 °C.

**13.** Method according to any one of claims 10 to 12, wherein the gold nanoparticle precursor is an aqueous $HAuCl_4$ solution and the combining of step c) is performed by immersing the precursor hydrogel in the aqueous $HAuCl_4$ solution.

**14.** Hydrogel obtainable by a method as defined in any one of claims 10 to 13.

**15.** Hydrogel as defined in any one of claims 1 to 8 or 14, or contact lens as defined in claim 9, for use in medicine, preferably in ophthalmology.

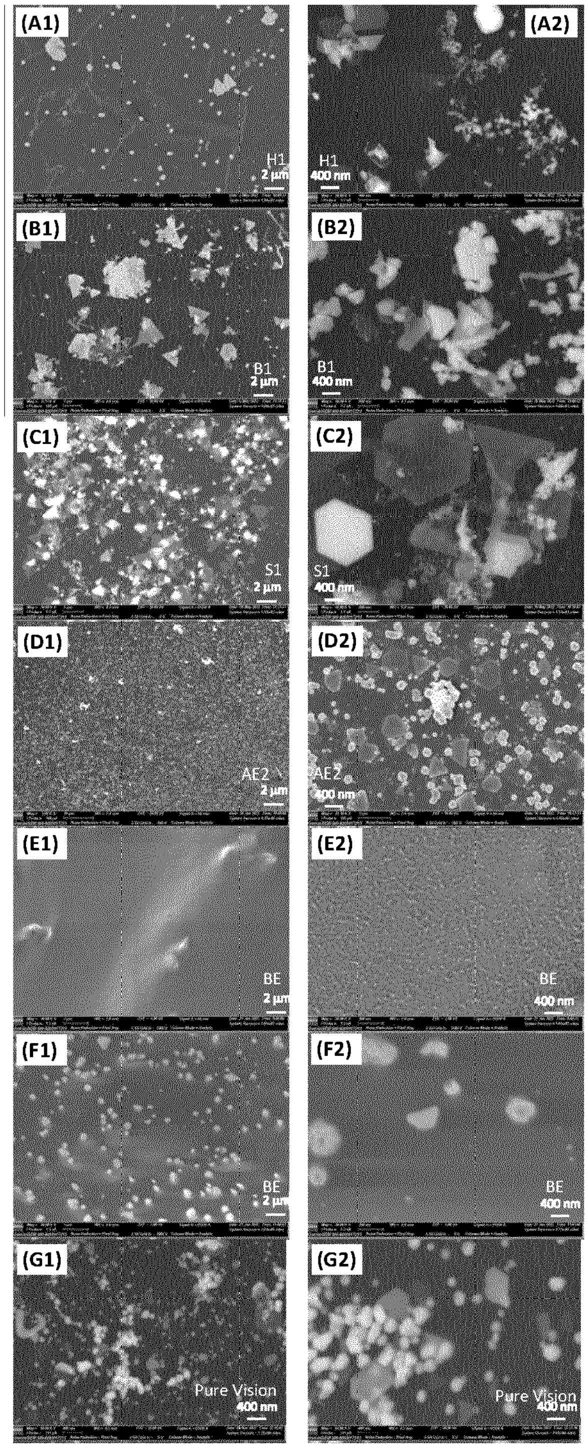
(A1)
H1
2 µm
(A2)
H1
400 nm
(B1)
B1
2 µm
(B2)
B1
400 nm
(C1)
S1
2 µm
(C2)
S1
400 nm
(D1)
AE2
2 µm
(D2)
AE2
400 nm
(E1)
BE
(E2)
BE
400 nm
(F1)
BE
(F2)
BE
400 nm
(G1)
Pure Vision
400 nm
(G2)
Pure Vision
400 nm

Figure 1

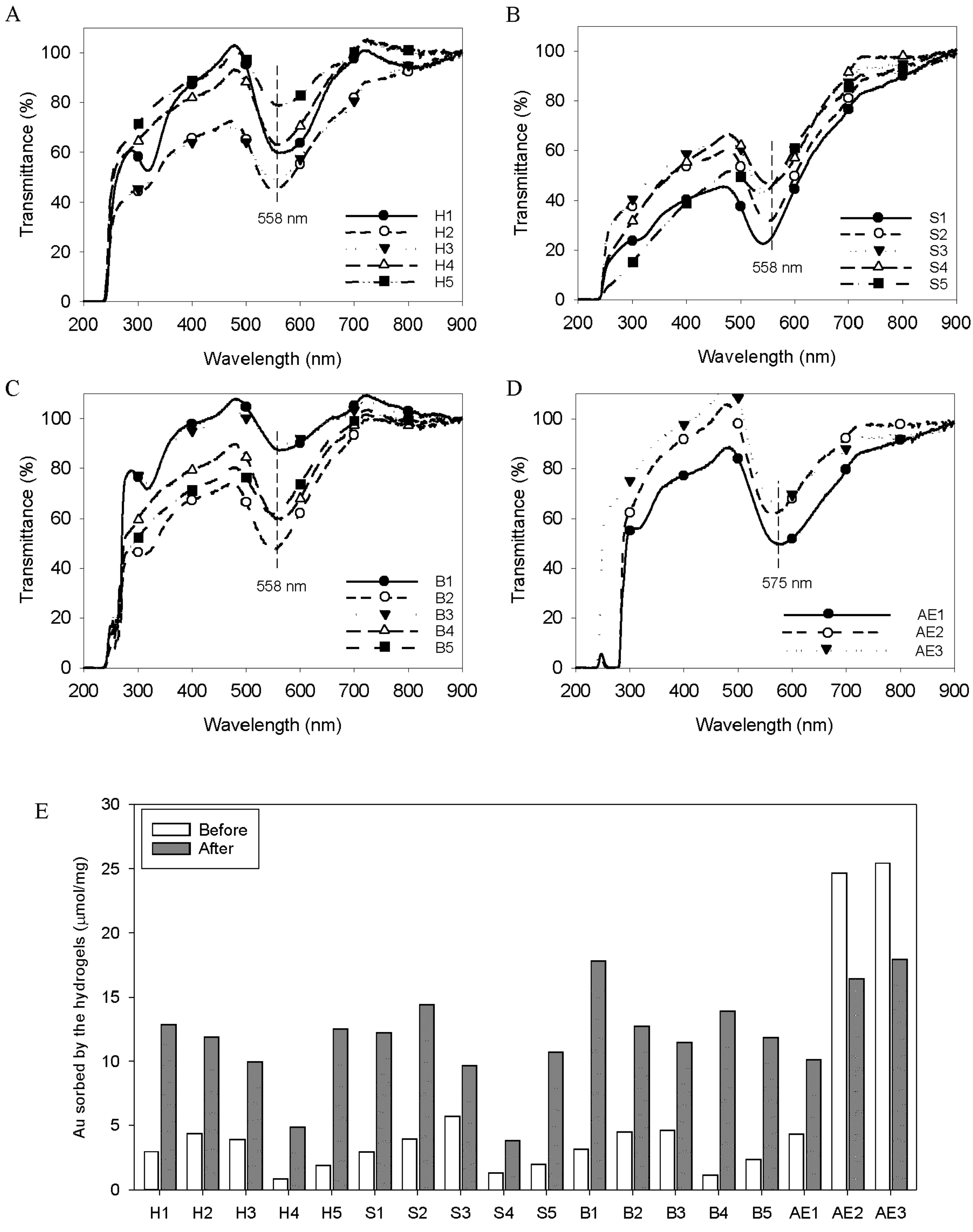
A
Transmittance (%)
Wavelength (nm)
558 nm
H1
H2
H3
H4
H5
B
S1
S2
S3
S4
S5
C
B1
B2
B3
B4
B5
D
575 nm
AE1
AE2
AE3
E
Au sorbed by the hydrogels (μmol/mg)
Before
After
H1 H2 H3 H4 H5 S1 S2 S3 S4 S5 B1 B2 B3 B4 B5 AE1 AE2 AE3

Figure 2

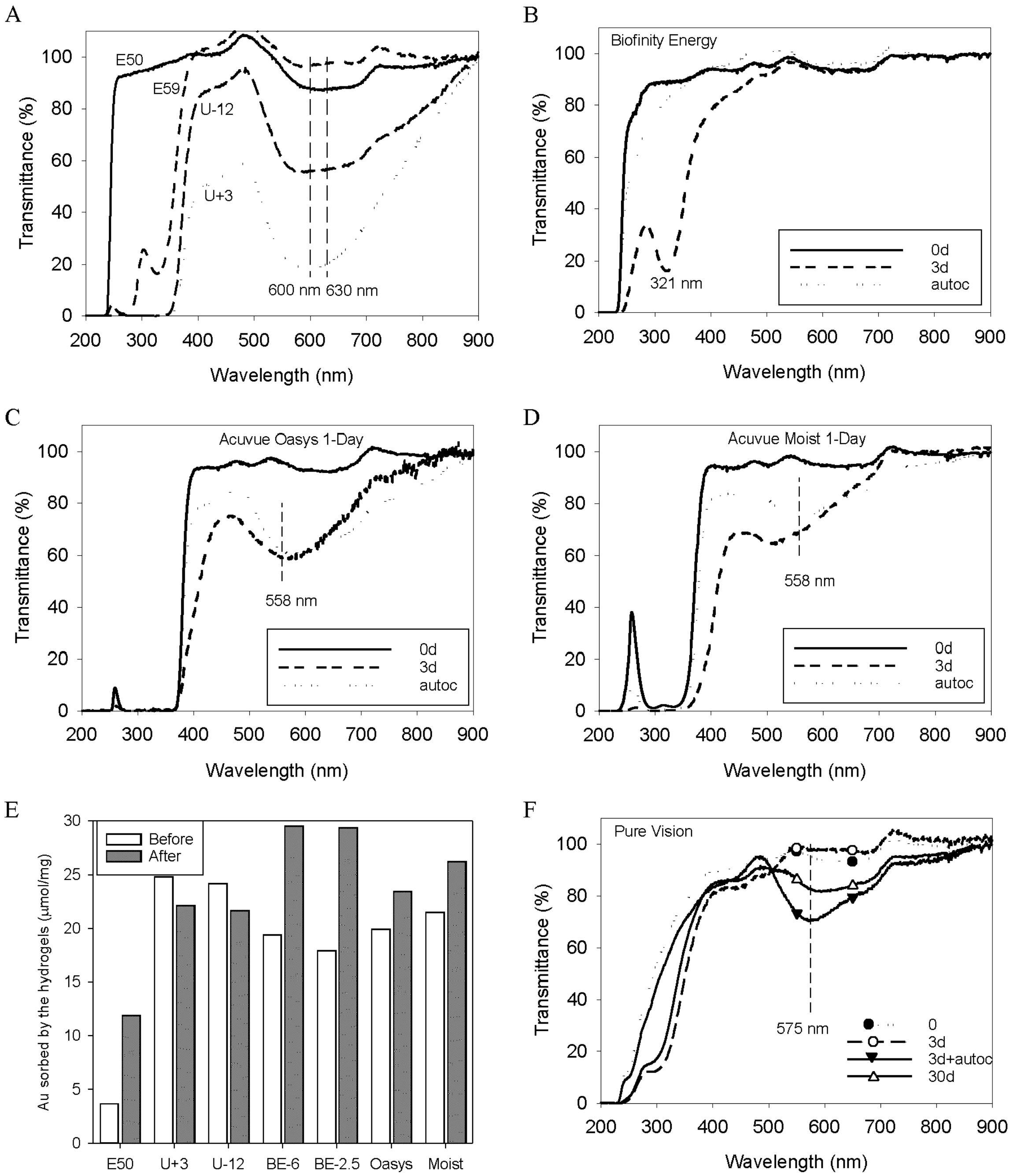

A
E50
E59
U-12
U+3
600 nm 630 nm
Transmittance (%)
Wavelength (nm)
B
Biofinity Energy
321 nm
0d
3d
autoc
Transmittance (%)
Wavelength (nm)
C
Acuvue Oasys 1-Day
558 nm
0d
3d
autoc
Transmittance (%)
Wavelength (nm)
D
Acuvue Moist 1-Day
558 nm
0d
3d
autoc
Transmittance (%)
Wavelength (nm)
E
Before
After
Au sorbed by the hydrogels (µmol/mg)
E50
U+3
U-12
BE-6
BE-2.5
Oasys
Moist
F
Pure Vision
575 nm
0
3d
3d+autoc
30d
Transmittance (%)
Wavelength (nm)


Figure 3

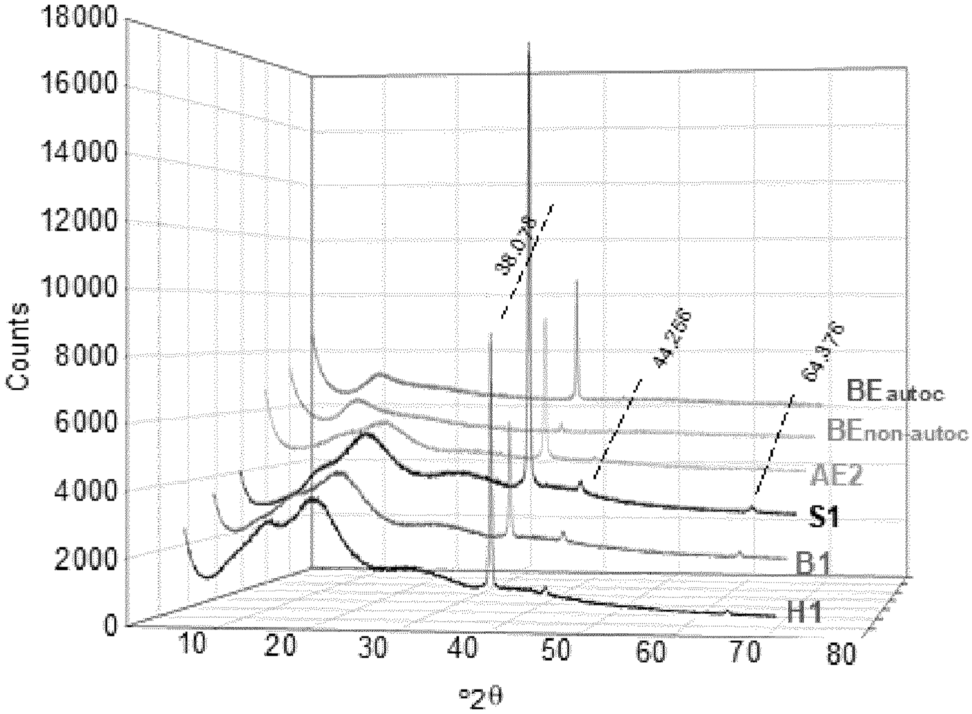
18000
16000
14000
12000
10000
8000
6000
4000
2000
0
Counts
38.078
44.266
64.376
BEautoc
BEnon-autoc
AE2
S1
B1
H1
10
20
30
40
50
60
70
80
°2θ

Figure 4

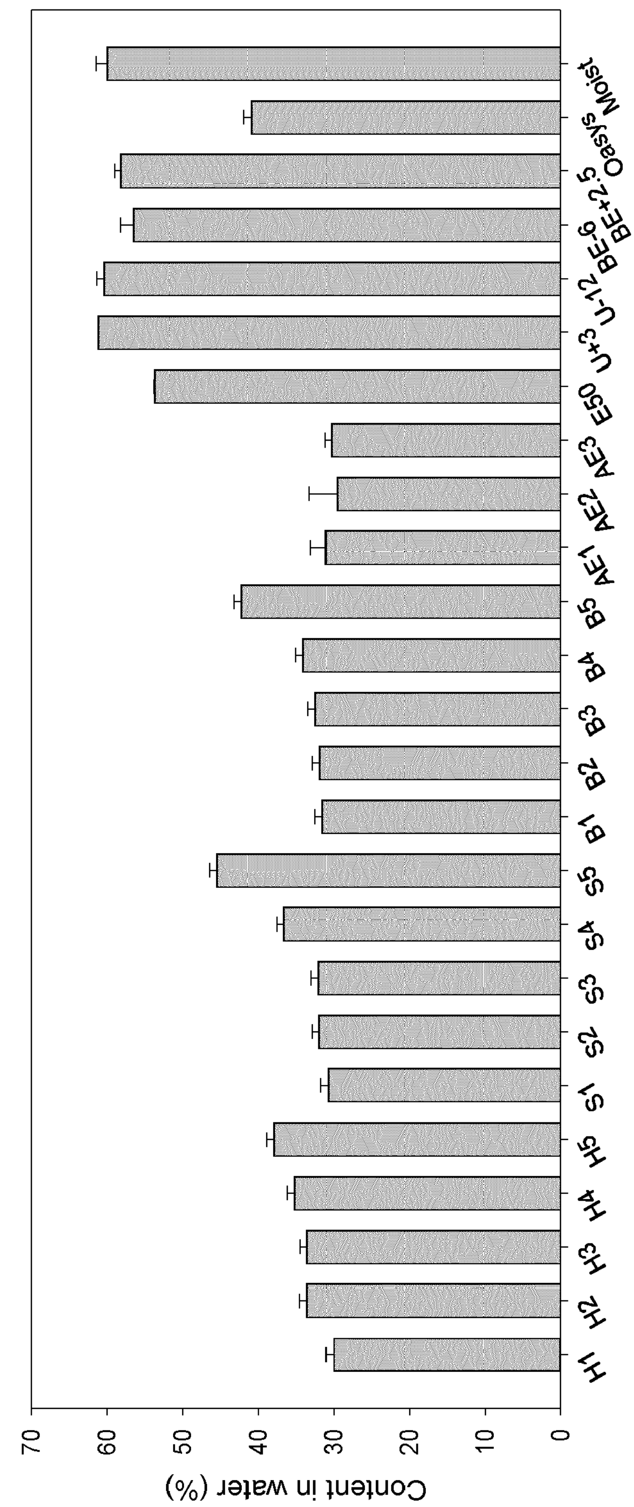
Content in water (%)
0
10
20
30
40
50
60
70
H1
H2
H3
H4
H5
S1
S2
S3
S4
S5
B1
B2
B3
B4
B5
AE1
AE2
AE3
E50
U+3
U-12
BE-6
BE+2.5
Oasys
Moist

Figure 5

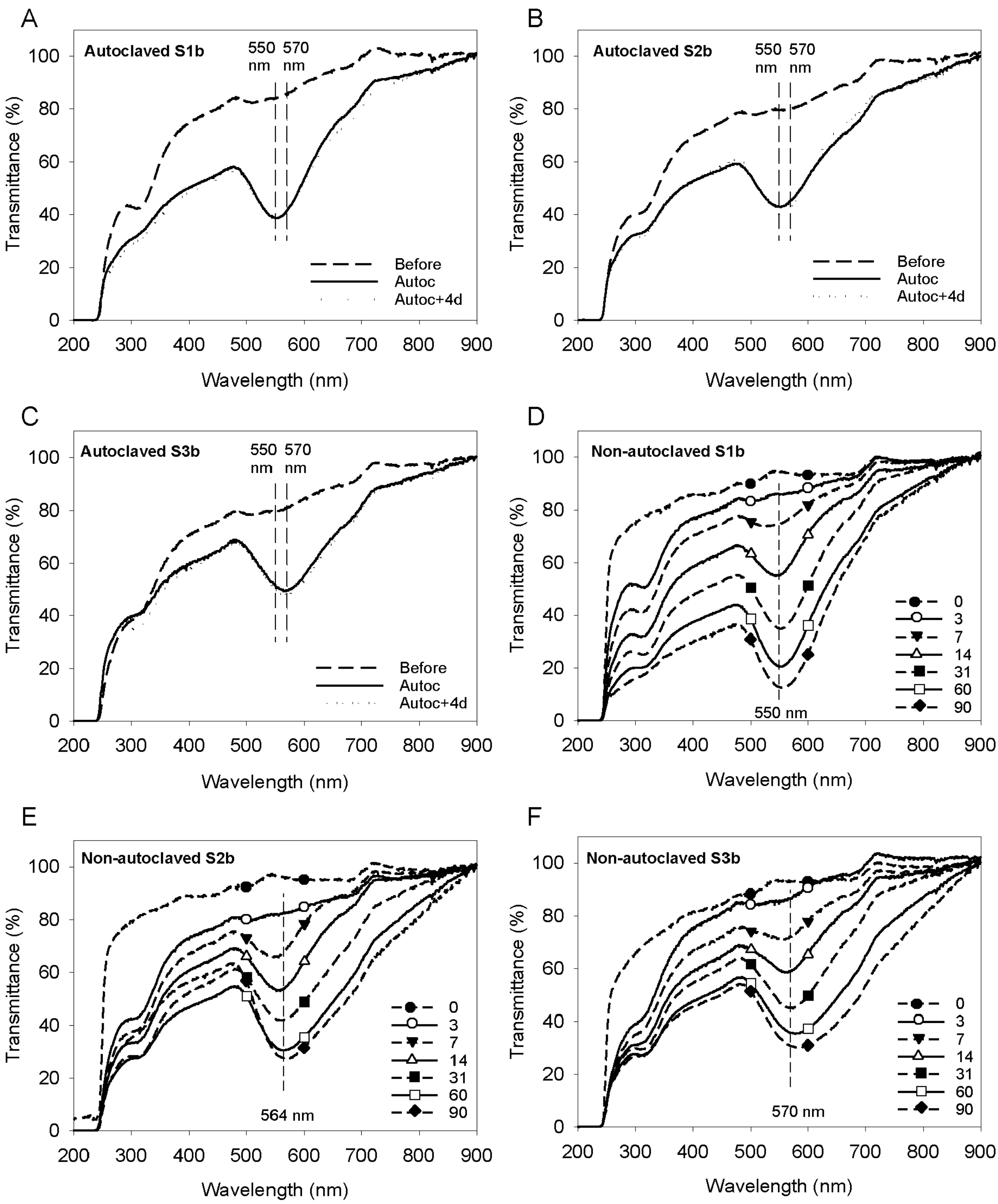
A
Autoclaved S1b
550 nm
570 nm
Before
Autoc
Autoc+4d
B
Autoclaved S2b
550 nm
570 nm
Before
Autoc
Autoc+4d
C
Autoclaved S3b
550 nm
570 nm
Before
Autoc
Autoc+4d
D
Non-autoclaved S1b
550 nm
0
3
7
14
31
60
90
E
Non-autoclaved S2b
564 nm
0
3
7
14
31
60
90
F
Non-autoclaved S3b
570 nm
0
3
7
14
31
60
90
Transmittance (%)
Wavelength (nm)
0
20
40
60
80
100
200
300
400
500
600
700
800
900

Figure 6

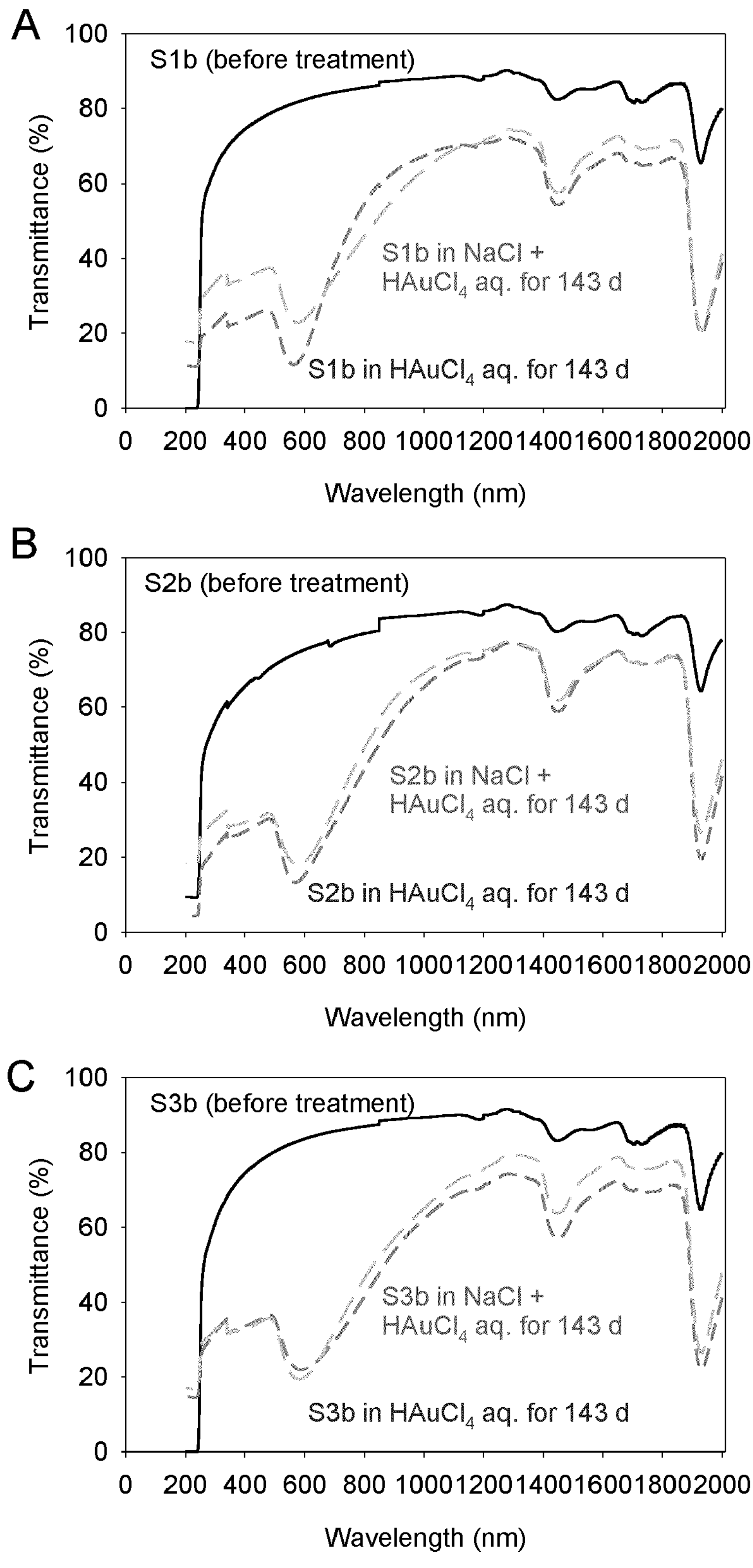
A
B
C
S1b (before treatment)
S1b in NaCl + $HAuCl_4$ aq. for 143 d
S1b in $HAuCl_4$ aq. for 143 d
S2b (before treatment)
S2b in NaCl + $HAuCl_4$ aq. for 143 d
S2b in $HAuCl_4$ aq. for 143 d
S3b (before treatment)
S3b in NaCl + $HAuCl_4$ aq. for 143 d
S3b in $HAuCl_4$ aq. for 143 d
Transmittance (%)
Wavelength (nm)
0
20
40
60
80
100
0
200
400
600
800
1000
1200
1400
1600
1800
2000

Figure 7

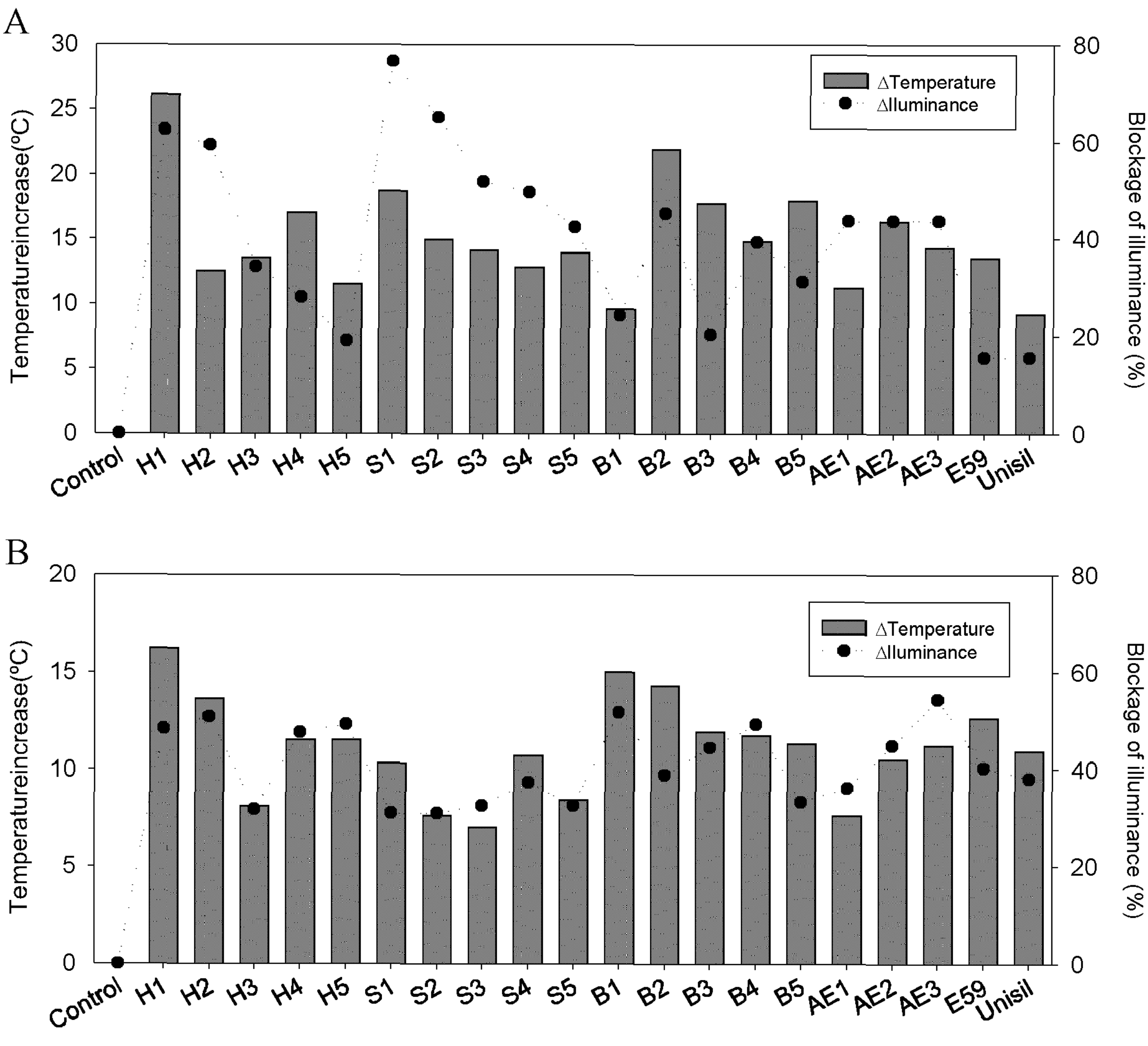
A
Temperatureincrease(ºC)
Blockage of illuminance (%)
ΔTemperature
ΔIlluminance
Control
H1
H2
H3
H4
H5
S1
S2
S3
S4
S5
B1
B2
B3
B4
B5
AE1
AE2
AE3
E59
Unisil
B
Temperatureincrease(ºC)
Blockage of illuminance (%)
ΔTemperature
ΔIlluminance
Control
H1
H2
H3
H4
H5
S1
S2
S3
S4
S5
B1
B2
B3
B4
B5
AE1
AE2
AE3
E59
Unisil

Figure 8

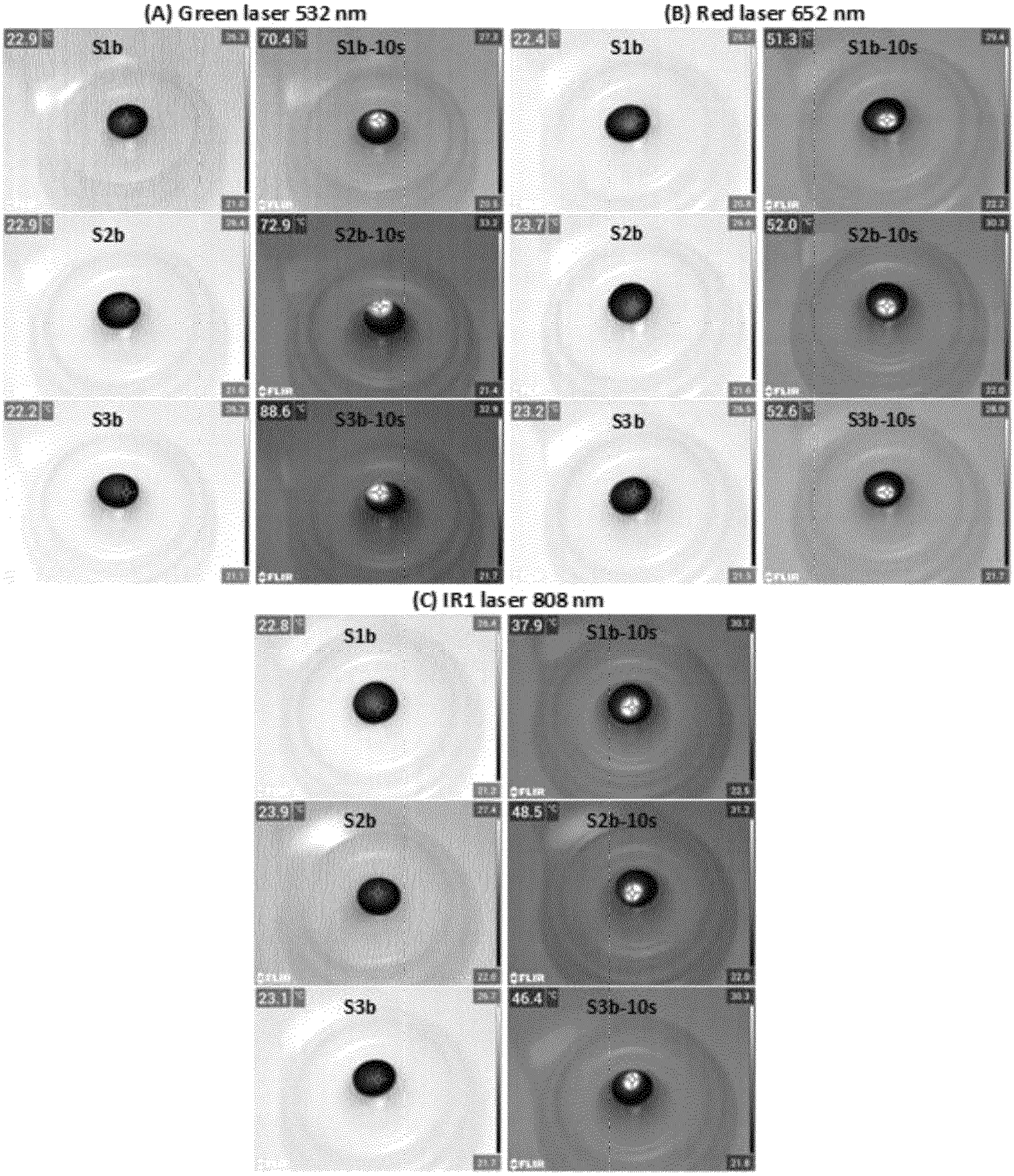
(A) Green laser 532 nm
(B) Red laser 652 nm
S1b
S1b-10s
S2b
S2b-10s
S3b
S3b-10s
22.9
70.4
22.4
51.3
22.9
72.9
23.7
52.0
22.2
88.6
23.2
52.6
(C) IR1 laser 808 nm
22.8
37.9
23.9
48.5
23.1
46.4

Figure 9 A-C

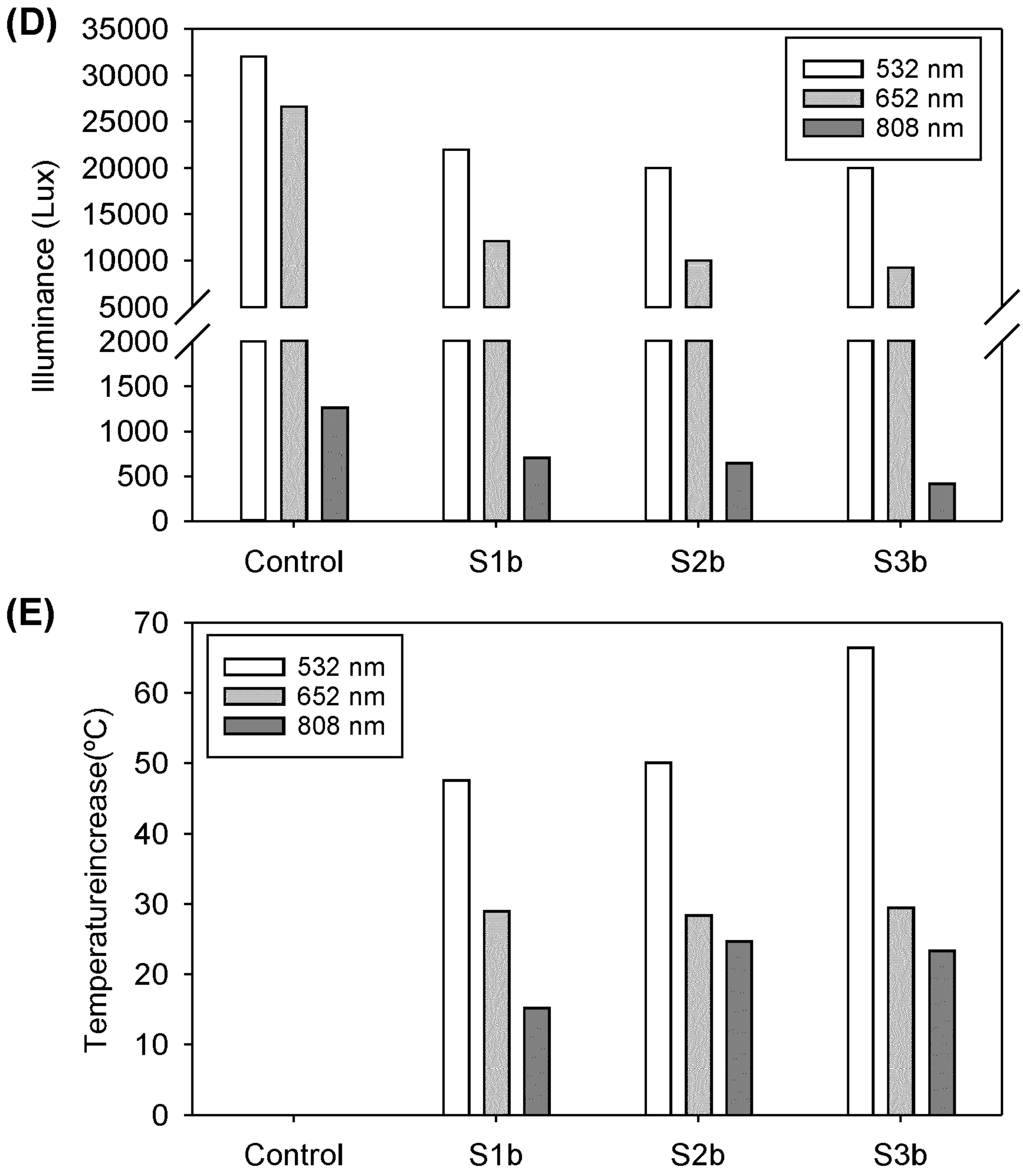
(D)
35000
30000
25000
20000
15000
10000
5000
2000
1500
1000
500
0
Illuminance (Lux)
532 nm
652 nm
808 nm
Control
S1b
S2b
S3b
(E)
70
60
50
40
30
20
10
0
Temperatureincrease(ºC)
532 nm
652 nm
808 nm
Control
S1b
S2b
S3b

Figure 9 D-E

Europäisches Patentamt
European Patent Office
Office européen des brevets

# EUROPEAN SEARCH REPORT

Application Number
EP 23 38 2049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SALIH AHMED E. ET AL: "Gold Nanocomposite Contact Lenses for Color Blindness Management", ACS NANO, vol. 15, no. 3, 11 February 2021 (2021-02-11), pages 4870-4880, XP093046942, US ISSN: 1936-0851, DOI: 10.1021/acsnano.0c09657 Retrieved from the Internet: URL:https://pubs.acs.org/doi/pdf/10.1021/acsnano.0c09657> | 1,9,15 | INV. A61K41/00 A61K47/69 A61P27/02 A61P27/04 |
| Y | * page 4877, column 2, paragraph 1; figure 6 * * page 4874; figure 3 * * page 4879 * ----- | 1-15 | |
| X | HYE HUN PARK ET AL: "Thermo- and pH-responsive hydrogel-coated gold nanoparticles prepared from rationally designed surface-confined initiators", JOURNAL OF NANOPARTICLE RESEARCH ; AN INTERDISCIPLINARY FORUM FOR NANOSCALE SCIENCE AND TECHNOLOGY, KLUWER ACADEMIC PUBLISHERS, DO, vol. 13, no. 7, 28 December 2010 (2010-12-28), pages 2909-2918, XP019917060, ISSN: 1572-896X, DOI: 10.1007/S11051-010-0180-3 | 1 | TECHNICAL FIELDS SEARCHED (IPC) A61K |
| Y | * page 2916; figure 6 * ----- | 1-15 | |
| Y | WO 2019/060704 A1 (UNIV FLORIDA [US]) 28 March 2019 (2019-03-28) * claims 11-23; examples 5, 6 * ----- -/-- | 1-15 | |

The present search report has been drawn up for all claims

| Place of search | Date of completion of the search | Examiner |
|---|---|---|
| The Hague | 11 July 2023 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

Europäisches Patentamt
European Patent Office
Office européen des brevets

# EUROPEAN SEARCH REPORT

Application Number
EP 23 38 2049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YING ZHOU ET AL: "Waveguiding Microactuators Based on a Photothermally Responsive Nanocomposite Hydrogel", ADVANCED FUNCTIONAL MATERIALS, WILEY - V C H VERLAG GMBH & CO. KGAA, DE, vol. 26, no. 30, 27 May 2016 (2016-05-27), pages 5447-5452, XP072409601, ISSN: 1616-301X, DOI: 10.1002/ADFM.201601569 * abstract * ----- | 1-15 | |
| Y | PEREIRA-DA-MOTA ANA F. ET AL: "Atorvastatin-Eluting Contact Lenses: Effects of Molecular Imprinting and Sterilization on Drug Loading and Release", PHARMACEUTICS, vol. 13, no. 5, 22 April 2021 (2021-04-22), page 606, XP093059181, DOI: 10.3390/pharmaceutics13050606 Retrieved from the Internet: URL:https://www.ncbi.nlm.nih.gov/pmc/articles/PMC8143582/pdf/pharmaceutics-13-00606.pdf> * the whole document * ----- | 1-15 | TECHNICAL FIELDS SEARCHED (IPC) |
| Y | LIU ZHEN ET AL: "Gold nanoparticles-loaded contact lenses for laser protection and Meibomian Gland Dysfunction (MGD) dry eye treatment", COLLOIDS AND SURFACES A : PHYSIOCHEMICAL AND ENGINEERINGS ASPECTS, vol. 635, 1 February 2022 (2022-02-01), page 128053, XP093059029, AMSTERDAM, NL ISSN: 0927-7757, DOI: 10.1016/j.colsurfa.2021.128053 * abstract * ----- -/-- | 1-15 | |

The present search report has been drawn up for all claims

| Place of search | Date of completion of the search | Examiner |
|---|---|---|
| The Hague | 11 July 2023 | Langer, Miren |

4

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 2 of 4

Europäisches Patentamt
European Patent Office
Office européen des brevets

# EUROPEAN SEARCH REPORT

Application Number
EP 23 38 2049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | LIU ZHEN ET AL: "Gold nanoparticle synthesis in contact lenses for drug-less ocular cystinosis treatment", EUROPEAN JOURNAL OF PHARMACEUTICS AND BIOPHARMACEUTICS, ELSEVIER SCIENCE PUBLISHERS B.V., AMSTERDAM, NL, vol. 165, 24 May 2021 (2021-05-24), pages 271-278, XP086615595, ISSN: 0939-6411, DOI: 10.1016/J.EJPB.2021.05.019 [retrieved on 2021-05-24] ----- | 1-15 | |
| A | HU XIAOPEI ET AL: "Multifunctional Gold Nanoparticles: A Novel Nanomaterial for Various Medical Applications and Biological Activities", FRONTIERS IN BIOENGINEERING AND BIOTECHNOLOGY, vol. 8, 13 August 2020 (2020-08-13), XP093059022, DOI: 10.3389/fbioe.2020.00990 Retrieved from the Internet: URL:http://dx.doi.org/10.3389/fbioe.2020.00990> ----- | 1-15 | |

-/--

TECHNICAL FIELDS SEARCHED (IPC)

The present search report has been drawn up for all claims

| Place of search | Date of completion of the search | Examiner |
|---|---|---|
| The Hague | 11 July 2023 | Langer, Miren |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 4

Europäisches Patentamt
European Patent Office
Office européen des brevets

# EUROPEAN SEARCH REPORT

Application Number
EP 23 38 2049

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | ALVAREZ-LORENZO CARMEN ET AL: "Contact lenses that transform gold into nanoparticles for prophylaxis of light-related events and photothermal therapy",<br>INTERNATIONAL JOURNAL OF PHARMACEUTICS, vol. 641, 1 June 2023 (2023-06-01), page 123048, XP093059178,<br>NL<br>ISSN: 0378-5173, DOI: 10.1016/j.ijpharm.2023.123048<br>Retrieved from the Internet:<br>URL:https://pdf.sciencedirectassets.com/271189/1-s2.0-S0378517323X00106/1-s2.0-S0378517323004684/main.pdf?X-Amz-Security-Token=IQoJb3JpZ2luX2VjECMaCXVzLWVhc3QtMSJHMEUCIQDX+rGIhpTVaCuBuIkLvGOgIzoqFzrkfE8juOF+YXNTDwIgIxP5nR2xXn4eUAR+RmIRmZcFdPBS2GqsM6Vr7RKLkmwqvAUIjP//////////ARAFGgwwNTkwMDM1NDY4NjUiDHUkj><br>----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 11 July 2023 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

4

# ANNEX TO THE EUROPEAN SEARCH REPORT ON EUROPEAN PATENT APPLICATION NO.

EP 23 38 2049

This annex lists the patent family members relating to the patent documents cited in the above-mentioned European search report. The members are as contained in the European Patent Office EDP file on
The European Patent Office is in no way liable for these particulars which are merely given for the purpose of information.

11-07-2023

| Patent document cited in search report | Publication date | Patent family member(s) | Publication date |
|---|---|---|---|
| WO 2019060704 A1 | 28-03-2019 | EP 3684309 A1 | 29-07-2020 |
| | | US 2020253867 A1 | 13-08-2020 |
| | | US 2022079877 A1 | 17-03-2022 |
| | | WO 2019060704 A1 | 28-03-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **LIU et al.** Gold nanoparticles-loaded contact lenses for laser protection and Meibomian Gland Dysfunction (MGD) dry eye treatment. *Colloids Surf A,* 2022, vol. 635, 128053
- **LIU et al.** Gold nanoparticle synthesis in contact lenses for drug-less ocular cystinosis treatment. *Eur. J. Pharm. Biopharm.,* 2021, vol. 165, 271-278
- Living Polymerization Routes to Siloxane Macromers and Higher Order Silicone Structures. **GOFF et al.** Progress in Silicones and Silicone-Modified Materials. vol. 5, 59-78
- **KARLGARD et al.** *Applied Surface Science,* 2004, vol. 230, 106-114
- **WOJNAR, L. et al.** Practical Guide to Image Analysis. *ASM International,* 2000, 157-160
- **PEREIRA-DA-MOTA et al.** Atorvastatin-Eluting Contact Lenses: Effects of Molecular Imprinting and Sterilization on Drug Loading and Release. *Pharmaceutics,* May 2021, vol. 13 (5), 606
- **SHARIQ et al.** Successful Synthesis of Gold Nanoparticles through Ultrasonic Spray Pyrolysis from a Gold(III) Nitrate Precursor and Their Interaction with a High Electron Beam. *Chemistry Open,* 2018, vol. 7 (7), 533-542
- **A.F. PEREIRA-DA-MOTA ; M. VIVERO-LOPEZ ; A. TOPETE ; A. P. SERRO ; A. CONCHEIRO ; C. ALVAREZ-LORENZO.** Atorvastatin-Eluting Contact Lenses: Effects of Molecular Imprinting and Sterilization on Drug Loading and Release. *Pharmaceutics,* 2021, vol. 13 (5), 606